# EUROPEAN PATENT SPECIFICATION

(11) **EP 1 339 725 B1**
(45) Date of publication and mention of the grant of the patent: **22.11.2006**
(21) Application number: 01987166.4
(22) Date of filing: 29.11.2001
(51) Int. Cl.: C07F 5/02, C07B 61/00, A61K 33/22, A61P 31/04

(54) **DNA METHYL TRANSFERASE INHIBITORS**
DNA METHYL TRANSFERASE INHIBITOREN
INHIBITEURS DES ADN METHYLTRANSFERASES

(30) Priority: 30.11.2000 US 250202 P
(43) Date of publication of application: 03.09.2003
(73) Proprietor: THE PENN STATE RESEARCH FOUNDATION, University Park, PA 16802-7000 (US)
(72) Inventor: BENKOVIC, Stephen, J., State College, PA 16803 (US); SHAPIRO, Lucille, Stanford, CA 94305 (US); BAKER, Stephen, J., Leicester LE2 6GN (GB); WAHNON, Daphne, C., Ottawa, Ontario K1V 0H2 (CA); WALL, Mark, Harleysville, PA 19438 (US); SHIER, Vincent, K., Arlington, Virginia 22202 (US); SCOTT, Charles, P., 223 South 10th STR.PHILADELPHIA PN 19107 (US); BABOVAL, Justin, State College, Pennsylvania 16801 (US)
(74) Representative: Grünecker, Kinkeldey, Stockmair & Schwanhäusser Anwaltssozietät
(86) International application number: PCT/US2001/045129
(87) International publication number: WO 2002/044184

(56) References cited:
- WO-A-00/44387
- WO-A-00/75142
- DE-A- 19 829 949
- FR-A- 2 071 171
- US-A- 3 485 864
- US-A- 4 713 346
- DATABASE CA [Online] CHEMICAL ABSTRACTS SERVICE, COLUMBUS, OHIO, US; HOHAUS, EBERHARD ET AL: "Mass spectrometric studies on boron chelates. Part IV. Fragmentations of diphenylboron chelates and monophenylboron chelates - mixture analysis" retrieved from STN Database accession no. 91:174350 XP002209944 & INT. J. MASS SPECTROM. ION PHYS. (1979), 31(1-2), 113-23 ,
- DATABASE CA [Online] CHEMICAL ABSTRACTS SERVICE, COLUMBUS, OHIO, US; YUAN, GUOZHENG ET AL: "Ligand substitution reaction of diarylboron chelates" retrieved from STN Database accession no. 114:207314 XP002209945 & WUJI HUAXUE XUEBAO (1990), 6(3), 314-18 ,
- HOHAUS ET AL: "Boron chelates and boron metal chelates. I. Boron chelates with chelating agents of the pyridine and quinoline series and their N-oxides" CHEMISCHE BERICHTE, VERLAG CHEMIE GMBH. WEINHEIM, DE, vol. 102, 1969, pages 4025-4031, XP002127342 ISSN: 0009-2940

## Description

### BACKGROUND OF THE INVENTION

### 1. Field of the Invention

The present invention relates to the field of antibiotics and particularly antibacterial compounds. The invention specifically relates to antibiotics targeted to DNA modification enzymes, in particular adenine DNA methyltransferases, that are the components of a broad variety of different bacterial pathogens including those that are essential for bacterial cell growth. The invention particularly provides pharmaceutical compositions and uses related to inhibitors or such adenine DNA methyltransferases having little or no inhibitory effects on cytosine methyltransferases, and hence having limited effects on eukaryotic, particularly mammalian, cells. Methods for preparing and using the adenine DNA methyltransferase inhibitors of the invention, and pharmaceutical compositions thereof, are also illustrated.

### 2. Background of the Invention

One hallmark of the modern era of medicine has been the decline in morbidity and mortality associated with bacterial infections. The development of a variety of antibiotic drugs in the early and middle parts of the twentieth century provided medical practitioners for the first time with effective treatments for a variety of infectious diseases.

However, misuse of conventional antibiotics and natural selection of the infectious bacterial population has resulted in the development of varying degrees of drug resistance by most bacterial infectious agents to most antibiotic agents. In severe cases, such as MRSA (Multidrug-Resistant StaphA), one or only a few antibiotics are currently effective. In addition, the existence of immunodeficiency syndromes results in additional incidence of opportunistic infections requiring intensive antibiotic treatment.

Thus, there is an increasing need in the art for novel, more effective antibiotic compounds for treating bacterial infections that are resistant to currently available therapies.

Most bacteria modify their genomic DNA by methylation of specific nucleotide bases. DNA methylation is critical to gene regulation and repair of mutational lesions *(see* Jost & Soluz, 1993, DNA METHYLATION, MOLECULAR BIOLOGY AND BIOLOGICAL SIGNIFICANCE, Birhauser Verlag: Basel, Switzerland; Palmer & Marinus, 1994, *Gene* 143: 1-12; Dryden, 1999, "Bacterial DNA Methyltransferases," in S-ADENOSYLMETHIONINE-DEPENDENT METHYLTRANSFERASES: STRUCTURES AND FUNCTIONS, X. Cheng and R. M. Blumenthal (eds.), World Scientific Publishing, p.283-340 *for review).* DNA methylation is catalyzed by a class of enzymes having different sequences specificities. There are those DNA methyltransferases for example (*dam*) that methylate adenine residues in GATC sequences, or cytosine *(dcm)* residues in CCAGG or CCTGG sequences that are not contained in the recognition site of a cognate restriction enzyme. There are those DNA methyltransferases that methylate residues contained in the recognition site of a cognate restriction enzyme *(for example, Apa*I*, Ava*II*, Bcl*I, *Cla*I*, Dpn*II, *Eco*RI, *Hae*III, *Hha*I*, Mbo*I, and *Msp*I*; see,* Marinus & Morris, 1973, *J. Bacteriol.* 114: 1143-1150; May & Hatman, 1975, *J. Bacteriol.* 123: 768-770; Heitman, 1993, *Genet. Eng.* 15: 57-108). In addition, the instant inventors have discovered an adenine DNA methyltransferase from *Caulobacter cresentus* that methylates the adenine residue in the sequence GANTC, as disclosed in International Application Publication No. WO98/12206. This methyltransferase is cell-cycle regulated and essential for successful bacterial cell growth; inhibition of the enzyme makes the bacteria non-viable. Similar methyltransferases have also been discovered in *Brucella abortus, Helicobacter pylori, Agrobacterium tumefaciens* and *Rhizobium meliloti.* In contrast with bacterial cells, DNA methylation in eukaryotic, and particularly mammalian cells, is limited to cytosine methylation at sites comprising the sequence CpG (Razin & Riggs, 1980, *Science* 210: 604-610; Jost & Bruhat, 1997, *Prog. Nucleic Acid Res*, *Molec. Biol.* 57: 217-248).

Thus, the existence of DNA methylation, in particular, the cell-cycle regulated adenine DNA methyltransferase found by the inventors in certain bacterial species, addresses the need in the art for novel targets for antibiotic activity.

### SUMMARY OF THE INVENTION

The invention provides pharmaceutical compositions comprising antibiotic compounds capable of inhibiting adenine DNA methyltransferases in bacterial cells. The antibiotic compounds of the invention specifically inhibit adenine-specific bacterial DNA methyltransferases, and do not inhibit bacterial or eukaryotic, particularly mammalian and most particularly human, cytosine-specific DNA methyltransferases. The compounds of the invention also inhibit adenine-specific DNA methyltransferases in plants. The antibiotic compounds are provided as pharmaceutical compositions capable of being administered to a human, for treatment of a disease having a bacterial etiology, or an opportunistic infection with a bacteria in a human, in an immunologically compromised or debilitated state of health.

The invention also illustrates methods for preparing the antibiotic compounds and pharmaceutical compositions thereof, and provides 2^{nd} med. uses of said antibiotics. Kits and packaged embodiments of the antibiotic compounds and pharmaceutical compositions of the invention are also disclosed.

Specific preferred embodiments of the present invention will become evident from the following more detailed description of certain preferred embodiments and the claims.

### DETAILED DESCRIPTION OF THE PREFERRED EMBODIMENTS

This invention provides pharmaceutical compositions comprising antibiotics, and specifically antibacterial compounds, that are inhibitors of bacterial adenine DNA methyltransferases. The compounds of the invention exhibit antibacterial, growth-inhibitory properties against any bacterial species that produces an adenine DNA methyltransferase. These include adenine DNA methyltransferases that are components of bacterial restriction/modification systems as understood in the art, as well as cell-cycle regulated adenine DNA methyltransferases (CcrM), such as those disclosed in International Application Publication No. WO98/12206.

US-A-3 485 864, FR-A-2071171, US-A-4 713 346 and Chemische Berichte, 102, 1969, 4025-4031 disclose single borinic esters apart from the pharmaceutical field.

WO00/44387 discloses the use of a borinic ester as an anti protozoal agent in the field of veterinary medicine.

WO00/75142 is a previous application of the present inventors and forms - in so far as it is relevant for the claimed subject matter of the present invention - only relevant prior art under Article 54(3) EPC. Thus, inhibitors of adenine DNA methyltransferases are particularly provided by the invention.

The adenine DNA methyltransferase inhibitors of the invention comprise a novel class of broad-spectrum antibiotics. Most bacterial species possess a DNA methyltransferase that is part of a modification apparatus, typically associated with a restriction enzyme, that preserves the integrity of cellular DNA while providing a defense against foreign (most typically viral) DNA. In addition, certain bacteria produce an adenine DNA methyltransferase that is essential for bacterial cell growth. Medically-important bacterial species that provide appropriate targets for the antibacterial activity of the inhibitors of the invention include gram-positive bacteria, including cocci such as *Staphylococcus* species and *Streptococcus* species; bacilli, including *Bacillus* species, *Corynebacterium* species and *Clostridium* species; filamentous bacteria, including *Actinomyces* species and *Streptomyces* species; gram-negative bacteria, including cocci such as *Neisseria* species; bacilli, such as *Pseudomonas* species, *Brucella* species, *Agrobacterium* species, *Bordetella* species, *Escherichia* species, *Shigella* species, *Yersinia* species, *Salmonella* species, *Klebsiella* species, *Enterobacter* species, *Hemophilus* species, *Pasteurella* species, and *Streptobacillus* species; spirochetal species, *Campylobacter* species, *Vibrio* species; and intracellular bacteria including *Rickettsiae* species and *Chlamydia* species.

Specific bacterial species that are targets for the adenine DNA methyltransferase inhibitors of the invention include *Staphylococcus aureus; Staphylococcus saprophyticus; Streptococcus pyrogenes; Streptococcus agalactiae; Streptococcus pneumoniae; Bacillus anthracis; Corynebacterium diphtheria; Clostridium perfringens; Clostridium botulinum; Clostridium tetani; Neisseria gonorrhoeae; Neisseria meningitidis; Pseudomonas aeruginosa; Legionella pneumophila; Escherichia coli; Yersinia pestis; Hemophilus influenzae; Helicobacter pylori; Campylobacter fetus; Vibrio cholerae; Vibrio parahemolyticus; Trepomena pallidum; Actinomyces israelii; Rickettsia prowazekii; Rickettsia rickettsii; Chlamydia trachomatis; Chlamydia psittaci; Brucella abortus* and *Agrobacterium tumefaciens.*

It is an important property of the adenine DNA methyltransferase inhibitors of the invention that the level of activity of these substances with cytosine-specific DNA methyltransferases is low. This is because cytosine-specific DNA methyltransferases occur in mammalian, most particularly human, cells, and it is an advantageous property of the adenine DNA methyltransferases of the invention to have little or no inhibitory activity against mammalian methyltransferases. This property confers upon the molecules provided by the invention the beneficial property of being bacterial cell specific, and having little antibiotic activity against mammalian, most preferably human, cells. Preferably, the IC₅₀ of these compounds for cytosine-specific DNA methyltransferases is greater than 500µM.

The invention also provides human pharmaceutical compositions comprising compounds of Formula I: 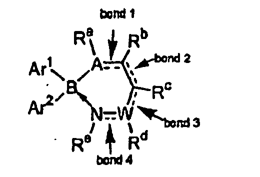 including all pharmaceutically acceptable salts of such compounds, and at least one pharmaceutically acceptable carrier or excipient;
wherein A is N, O or S;
W is Cₚ, where p is 0 or 1;
R^{a}, R^{b}, R^{c}, R^{d}, and R^{e} are the same or different and are independently hydrogen, halogen, lower alkyl, aryl or substituted aryl, lower alkoxy, lower alkoxyalkyl, or cycloalkyl or cycloalkyl alkoxy, where each cycloalkyl group has from 3-7 members, where up to two of the cycloalkyl members are optionally hetero atoms selected from sulfur, oxygen and nitrogen, and where any member of the alkyl, aryl or cycloalkyl group is optionally substituted with halogen, lower alkyl or lower alkoxy, aryl or substituted aryl, halogen, nitro, nitroso, aldehyde, carboxylic acid, amide, ester, or sulfate, or wherein R^{a}, R^{b}, R^{c}, R^{d}, and R^{e} may be connected by aromatic, aliphatic, heteroaromatic, heteroaliphatic ring structures or substituted embodiments thereof, where R^{a} is absent when A is O or S and R^{d} is absent when p = 0; and
wherein Ar¹ and Ar² can be the same or different and are each independently aryl or aryl substituted at one or a plurality of positions with halogen, nitro, nitroso, lower alkyl, aryl or substituted aryl, lower alkoxy, lower alkoxyalkyl, or cycloalkyl or cycloalkyl alkoxy, where each cycloalkyl group has from 3-7 members, where up to two of the cycloalkyl members are optionally hetero atoms selected from sulfur, oxygen and nitrogen, and where any member of the alkyl, aryl or cycloalkyl group is optionally substituted with halogen, lower alkyl or.lower alkoxy, aryl or substituted aryl, halogen, nitro, nitroso, aldehyde, carboxylic acid, amide, ester, or sulfate, and
optionally Ar¹ and Ar² may be cojoined to create a tricyclic scaffold. 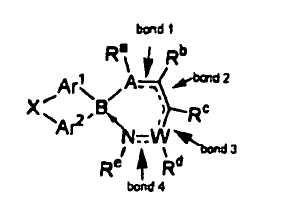 where X = C=O, CHOH, (CH₂)ₙ (n = 0 to 2), -CH=CH-, NR^{f} (R^{f} = H, C₁-C₄ alkyl, phenyl, thienyl, or pyridyl), O, SOₙ (n = 0 to 2), which have a plurality of positions with halogen, nitro, nitroso, lower alkyl, aryl or substituted aryl, lower alkoxy, lower alkoxyalkyl, or cycloalkyl or cycloalkyl alkoxy, where each cycloalkyl group has from 3-7 members, where up to two of the cycloalkyl members are optionally hetero atoms selected from sulfur, oxygen and nitrogen, and
wherein bond 1, bond 2, bond 3 and bond 4 are independently a single bond or a double bond, provided that when A is S or O, bond 1 is a single bond and where A is N, bond 1 is a double bond
with the proviso that if A is O, X is not present, and Ar¹ and Ar² are not cojoined, then p is not 0.

Thus, the invention provides pharmaceutical compositions comprising adenine DNA methyltransferase inhibitors that are derivatives of borinic acid, most preferably diphenyl or substituted diphenyl borinic acid, and most preferably diphenyl or substituted diphenyl borinic acid alkylamine esters thereof. In preferred embodiments, the compounds include or illustrate di-(4-fluorophenyl)borinic acid- 8-hydroxyquinoline ester, di-(4-chlorophenyl)borinic acid 8-hydroxyquinoline ester, di-(3-chlorophenyl)borinic acid 8-hydroxyquinoline ester, di-(4-chloro-2-fluorophenyl)borinic acid 8-hydroxyquinoline ester, di-(3,4-methylenedioxyphenyl)borinic acid 8-hydroxyquinoline ester, di-(4-methoxyphenyl)borinic acid 8-hydroxyquinoline ester, di-(2-thienyl)borinic acid 8-hydroxyquinoline ester, di-(p-fluorophenyl)borinic acid 8-hydroxyquinaldine ester, di-(p-chlorophenyl)borinic acid 8-hydroxyquinaldine ester, di-(4-methoxyphenyl)borinic acid 8-hydroxyquinaldine ester, di-(p-fluorophenyl)borinic acid 5-chloro-8-hydroxyquinaline ester, di-(p-chlorophenyl)borinic acid 5-chloro-8-hydroxyquinaline ester, di-(3,4-methylenedioxyphenyl) borinic acid 5-chloro-8-hydroxyquinoline ester, di-(4-methoxyphenyl)borinic acid 5-chloro-8-hydroxyquinoline ester, di-(3,4-methylenedioxyphenyl)borinic acid 8-hydroxy-5-nitroquinoline ester, diphenylborinic acid 2-aminophenol, diphenylborinic acid pyridine-2-raethanol, diphenylborinic acid 2-amino-1-phenylpropanol, diphenylborinic acid (S)-(+)-pyrrolidine-2-methanol, di-(4-fluorophenyl)borinic acid ethanolamine ester, and di-(4-chlorophenyl)boririic acid ethanolamine ester.

In certain situations, compounds of the pharmaceutical compositions of the invention may contain one or more asymmetric carbon atoms, so that the compounds can exist in different stereoisomeric forms. These compounds can be, for example, racemates or optically active forms. In these situations, the single enantiomers, *i.e.,* optically active forms, can be obtained by asymmetric synthesis or by resolution of the racemates. Resolution of the racemates can be accomplished, for example, by conventional methods such as crystallization in the presence of a resolving agent, or chromatography, using, for example a chiral HPLC column.

Regardless of how a putative adenine DNA methyltransferase is prepared according to the invention, the compound is analyzed for both adenine and cytosine-specific DNA methyltransferase activity. Susceptible bacteria (known to express an adenine DNA methyltransferase) are grown in the presence and absence of the inhibitory compound, and the extent of growth inhibition in the presence of the compound is determined relative to growth in the absence of the compound. The mechanism of action (*i.e.*, inhibition of adenine DNA methyltransferase) is verified for each growth-inhibitory compound by filter-binding radioassay using hemimethylated DNA, tritiated *S*-adenosyl methionine (C³H₃) and a purified adenine DNA methyltransferase according to International Application Publication No. WO98/12206.

Compounds of the pharmaceutical compositions of the invention can exist as tautomers in solution. When structures and names are given for one tautomeric form the other tautomeric form is also included in the invention.

Representative compounds include, but are not limited to the compounds disclosed herein and their pharmaceutically acceptable acid and base addition salts. In addition, if the compound is obtained as an acid addition salt, the free base can be obtained by basifying a solution of the acid salt Conversely, if the product is a free base, an addition salt, particularly a pharmaceutically acceptable addition salt, may be produced by dissolving the free base in a suitable organic solvent and treating the solution with an acid, in accordance with conventional procedures for preparing acid addition salts from base compounds.

The present invention also encompasses the acylated prodrugs of the compounds of the invention. Those skilled in the are will recognize various synthetic methodologies which may be employed to prepare non-toxic pharmaceutically acceptable addition salts and acylated prodrugs of the inventive compounds.

By "alkyl", "lower alkyl", and "C₁-C₆ alkyl in the present invention is meant straight or branched chain alkyl groups having 1-6 carbon atoms, such as, methyl, ethyl, propyl, isopropyl, n-butyl, *sec-*butyl, *tert*-butyl, pentyl, 2-pentyl, isopentyl, neopentyl, hexyl, 2-hexyl, 3-hexyl, and 3-methylpentyl.

By "alkoxy", "lower alkoxy", and "C₁-C₆ alkoxy" in the present invention is meant straight or branched chain alkoxy groups having 1-6 carbon atoms, such as, for example, methoxy, ethoxy, propoxy, isopropoxy, n-butoxy, sec-butoxy, *tert-*butoxy, pentoxy, 2-pentyl, isopentoxy, neopentoxy, hexoxy, 2-hexoxy, 3-hexoxy, and 3-methylpentoxy.

By the term "halogen" in the present invention is meant fluorine, bromine, chlorine, and iodine.

By "cycloalkyl", *e.g*., C₃-C₇ cycloalkyl, in the present invention is meant cycloalkyl groups having 3-7 atoms such as, for example cyclopropyl, cyclobutyl, cyclopentyl, cyclohexyl, and cycloheptyl. In the C₃-C₇ cycloalkyl groups, preferably in the C₅-C₇ cycloalkyl groups, one or two of the carbon atoms forming the ring can optionally be replaced with a hetero atom, such as sulfur, oxygen or nitrogen. Examples of such groups are piperidinyl, piperazinyl, morpholinyl, pyrrolidinyl, imidazolidinyl, oxazolidinyl, azaperhydroepinyl, oxazaperhydroepinyl, oxepanyl, oxazaperhydroinyl, and oxadiazaperhydroinyl. C₃ and C₄ cycloalkyl groups having a member replaced by nitrogen or oxygen include aziridinyl, azetidinyl, oxetanyl, and oxiranyl.

By "aryl" is meant an aromatic carbocyclic group having a single ring (e.g., phenyl), multiple rings (e.g., biphenyl), or multiple condensed rings in which at least one is aromatic, (e.g., 1,2,3,4-tetrahydronaphthyl, naphthyl, anthryl, or phenanthryl), which is optionally mono-, di-, or trisubstituted with, e.g., halogen, lower alkyl, lower alkoxy, lower alkylthio, trifluoromethyl, lower acyloxy, aryl, heteroaryl, and hydroxy. Preferred aryl groups include phenyl and naphthyl, each of which is optionally substituted as defined herein.

By "heteroaryl" is meant one or more aromatic ring systems of 5-, 6-, or 7-membered rings containing at least one and up to four heteroatoms selected from nitrogen, oxygen, or sulfur. Such heteroaryl groups include, for example, thienyl, furanyl, thiazolyl, imidazolyl, (is)oxazolyl, pyridyl, pyrimidinyl, (iso)quinolinyl, napthyridinyl, benzimidazolyl, benzoxazolyl. Preferred heteroaryls are thiazolyl, pyrimidinyl, preferrably pyrimidin-2-yl, and pyridyl. Other preferred heteroaryl groups include 1-imidazolyl, 2-thienyl, 1-, or 2- quinolinyl, 1-, or 2- isoquinolinyl, 1-, or 2- tetrahydro isoquinolinyl, 2- or 3- furanyl and 2- tetrahydrofuranyl.

The bacterial growth inhibitory, adenine DNA methyltransferase inhibiting compounds are described herein.

### Uses of the Compounds of the Invention

The invention provides embodiments of the compounds disclosed herein as pharmaceutical compositions. The pharmaceutical compositions of the present invention can be manufactured in a manner that is itself known, e.g., by means of a conventional mixing, dissolving, granulating, dragee-making, levigating, emulsifying, encapsulating, entrapping or lyophilizing processes.

Pharmaceutical compositions for use in accordance with the present invention thus can be formulated in conventional manner using one or more physiologically acceptable carriers comprising excipients and auxiliaries that facilitate processing of the active compounds into preparations that can be used pharmaceutically. Proper formulation is dependent upon the route of administration chosen.

Non-toxic pharmaceutical salts include salts of acids such as hydrochloric, phosphoric, hydrobromic, sulfuric, sulfinic, formic, toluenesulfonic, methanesulfonic, nitic, benzoic, citric, tartaric, maleic, hydroiodic, alkanoic such as acetic, HOOC-(CH₂)ₙ-CH₃ where n is 0-4, and the like. Non-toxic pharmaceutical base addition salts include salts of bases such as sodium, potassium, calcium, ammonium, and the like. Those skilled in the art will recognize a wide variety of non-toxic pharmaceutically acceptable addition salts.

For injection, the pharmaceutical compositions of the invention can be formulated in appropriate aqueous solutions, such as physiologically compatible buffers such as Hanks's solution, Ringer's solution, or physiological saline buffer. For transmucosal and transcutaneous administration, penetrants appropriate to the barrier to be permeated are used in the formulation. Such penetrants are generally known in the art.

For oral administration, the pharmaceutical compositions can be formulated readily by combining the active compounds with pharmaceutically acceptable carriers well known in the art. Such carriers enable the compounds of the invention to be formulated as tablets, pills, dragees, capsules, liquids, gels, syrups, slurries, suspensions and the like, for oral ingestion by a patient to be treated. Pharmaceutical preparations for oral use can be obtained with solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethylcellulose, sodium carboxymethylcellulose, and/or polyvinylpyrrolidone (PVP). If desired, disintegrating agents can be added, such as the cross-linked polyvinyl pyrrolidone, agar, or alginic acid or a salt thereof such as sodium alginate.

Dragee cores are provided with suitable coatings. For this purpose, concentrated sugar solutions can be used, which can optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments can be added to the tablets or dragee coatings for identification or to characterize different combinations of active compound doses.

Pharmaceutical preparations that can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds can be dissolved or suspended in suitable liquids, such as fatty oils, liquid paraffin, or liquid polyethylene glycols. In addition, stabilizers can be added. All formulations for oral administration should be in dosages suitable for such administration. For buccal administration, the compositions can take the form of tablets or lozenges formulated in conventional manner.

For administration by inhalation, the pharmaceutical compositions for use according to the present invention are conveniently delivered in the form of an aerosol spray presentation from pressurized packs or a nebuliser, with the use of a suitable propellant, *e.g*., dichlorodifluoromethane, trichlorofluoromethane, dichlorotetrafluoroethane, carbon dioxide or other suitable gas. In the case of a pressurized aerosol the dosage unit can be determined by providing a valve to deliver a metered amount. Capsules and cartridges of *e.g ,* gelatin for use in an inhaler or insufflator can be formulated containing a powder mix of the compound and a suitable powder base such as lactose or starch.

The pharmaceutical compositions can be formulated for parenteral administration by injection, e.g., by bolus injection or continuous infusion. Formulations for injection can be presented in unit dosage form, *e.g*., in ampoules or in multi-dose containers, with an added preservative. The compositions can take such forms as suspensions, solutions or emulsions in oily or aqueous vehicles, and can contain formulatory agents such as suspending, stabilizing and/or dispersing agents.

Pharmaceutical formulations for parenteral administration include aqueous solutions of the active compounds in water-soluble form. Additionally, suspensions of the active compounds can be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions can contain substances that increase the viscosity of the suspension, such as sodium carboxymethyl cellulose, sorbitol, or dextran. Optionally, the suspension can also contain suitable stabilizers or agents that increase the solubility of the compounds to allow for the preparation of highly concentrated solutions. Alternatively, the active ingredient can be in powder form for constitution with a suitable vehicle, *e.g*., sterile pyrogen-free water, before use. The compounds can also be formulated in rectal compositions such as suppositories or retention enemas, *e.g*., containing conventional suppository bases such as cocoa butter or other glycerides.

In addition to the formulations described previously, the compounds can also be formulated as a depot preparation. Such long acting formulations can be administered by implantation (for example subcutaneously or intramuscularly) or by intramuscular injection. Thus, for example, the compounds can be formulated with suitable polymeric or hydrophobic materials (for example as an emulsion in an acceptable oil) or ion exchange resins, or as sparingly soluble derivatives, for example, as a sparingly soluble salt.

A pharmaceutical carrier for the hydrophobic compounds of the invention is a cosolvent system comprising benzyl alcohol, a nonpolar surfactant, a water-miscible organic polymer, and an aqueous phase. The cosolvent system can be the VPD co-solvent system. VPD is a solution of 3% w/v benzyl alcohol, 8% w/v of the nonpolar surfactant polysorbate 80, and 65% w/v polyethylene glycol 300, made up to volume in absolute ethanol. The VPD co-solvent system (VPD:5W) consists of VPD diluted 1:1 with a 5% dextrose in water solution. This co-solvent system dissolves hydrophobic compounds well, and itself produces low toxicity upon systemic administration. Naturally, the proportions of a co-solvent system can be varied considerably without destroying its solubility and toxicity characteristics. Furthermore, the identity of the co-solvent components can be varied: for example, other low-toxicity nonpolar surfactants can be used instead of polysorbate 80; the fraction size of polyethylene glycol can be varied; other biocompatible polymers can replace polyethylene glycol, *e.g*. polyvinyl pyrrolidone; and other sugars or polysaccharides can substitute for dextrose.

Alternatively, other delivery systems for hydrophobic pharmaceutical compounds can be employed. Liposomes and emulsions are well known examples of delivery vehicles or carriers for hydrophobic drugs. Certain organic solvents such as dimethylsulfoxide also can be employed, although usually at the cost of greater toxicity. Additionally, the compounds can be delivered using a sustained-release system, such as semipermeable matrices of solid hydrophobic polymers containing the therapeutic agent. Various sustained-release materials have been established and are well known by those skilled in the art. Sustained-release capsules can, depending on their chemical nature, release the compounds for a few weeks up to over 100 days. Depending on the chemical nature and the biological stability of the therapeutic reagent, additional strategies for protein and nucleic acid stabilization can be employed.

The pharmaceutical compositions also can comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

The pharmaceutical compositions of the invention can be provided as salts with pharmaceutically compatible counterions. Pharmaceutically compatible salts can be formed with many acids, including but not limited to hydrochloric, sulfuric, acetic, lactic, tartaric, malic, succinic, phosphoric, hydrobromic, sulfinic, formic, toluenesulfonic, methanesulfonic, nitic, benzoic, citric, tartaric, maleic, hydroiodic, alkanoic such as acetic, HOOC-(CH₂)ₙ-CH₃ where n is 0-4, and the like. Salts tend to be more soluble in aqueous or other protonic solvents that are the corresponding free base forms. Non-toxic pharmaceutical base addition salts include salts of bases such as sodium, potassium, calcium, ammonium, and the like. Those skilled in the art will recognize a wide variety of non-toxic pharmaceutically acceptable addition salts.

Pharmaceutical compositions of the compounds of the present invention can be formulated and administered through a variety of means, including systemic, localized, or topical administration. Techniques for formulation and administration can be found in "Remington's Pharmaceutical Sciences," Mack. Publishing Co., Easton, PA. The mode of administration can be selected to maximize delivery to a desired target site in the body. Suitable routes of administration can, for example, include oral, rectal, transmucosal, transcutaneous, or intestinal administration; parenteral delivery, including intramuscular, subcutaneous, intramedullary injections, as well as intrathecal, direct intraventricular, intravenous, intraperitoneal, intranasal, or intraocular injections.

Alternatively, one can administer the compound in a local rather than systemic manner, for example, *via* injection of the compound directly into a specific tissue, often in a depot or sustained release formulation.

Pharmaceutical compositions suitable for use in the present invention include compositions wherein the active ingredients are contained in an effective amount to achieve its intended purpose. More specifically, a therapeutically effective amount means an amount effective to prevent development of or to alleviate the, existing symptoms of the subject being treated. Determination of the effective amounts is well within the capability of those skilled in the art, especially in light of the detailed disclosure provided herein.

For any compound used in the uses of the invention, the therapeutically effective dose can be estimated initially from cell culture assays, as disclosed herein. For example, a dose can be formulated in animal models to achieve a circulating concentration range that includes the EC50 (effective, dose for 50% increase) as determined in cell culture, *i.e.,* the concentration of the test compound which achieves a half-maximal inhibition of bacterial cell growth. Such information can be used to more accurately determine useful doses in humans.

It will be understood, however, that the specific dose level for any particular patient will depend upon a variety of factors including the activity of the specific compound employed, the age, body weight, general health, sex, diet, time of administration, route of administration, and rate of excretion, drug combination, the severity of the particular disease undergoing therapy and the judgment of the prescribing physician.

Preferred compounds will have certain pharmacological properties. Such properties include, but are not limited to oral bioavailability, low toxicity, low serum protein binding and desirable *in vitro* and *in vivo* half-lives. Assays may be used to predict these desirable pharmacological properties. Assays used to predict bioavailability include transport across human intestinal cell monolayers, including Caco-2 cell monolayers. Serum protein binding may be predicted from albumin binding assays. Such assays are described in a review by Oravcová *et al*. (1996, *J. Chromat. B* 677: 1-27). Compound half-life is inversely proportional to the frequency of dosage of a compound. *In vitro* half-lives of compounds may be predicted from assays of microsomal half-life as described by Kuhnz and Gieschen (Drug Metabolism and Disposition, (1998) volume 26, pages 1120-1127).

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio between LD50 and ED50. Compounds that exhibit high therapeutic indices are preferred. The data obtained from these cell culture assays and animal studies can be used in formulating a range of dosage for use in humans. The dosage of such compounds lies preferably within a range of circulating concentrations that include the ED50 with little or no toxicity. The dosage can vary within this range depending upon the dosage form employed and the route of administration utilized. The exact formulation, route of administration and dosage can be chosen by the individual physician in view of the patient's condition. (*See*, *e.g.* Fingl *et al.,* 1975, in "The Pharmacological Basis of Therapeutics", Ch.1, p.1).

Dosage amount and interval can be adjusted individually to provide plasma levels of the active moiety that are sufficient to maintain bacterial cell growth inhibitory effects. Usual patient dosages for systemic administration range from 100 - 2000 mg/day. Stated in terms of patient body surface areas, usual dosages range from 50 - 910 mg/m²/day. Usual average plasma levels should be maintained within 0.1-1000 µM. In cases of local administration or selective uptake, the effective local concentration of the compound cannot be related to plasma concentration.

The compounds of the pharmaceutical compositions of the invention are modulators of cellular processes in bacteria that infect plants, animals and humans. The pharmaceutical compositions of the adenine DNA methyltransferase inhibitory compounds.of the invention are useful as antibiotics for the treatment of diseases of both animals and humans, including but not limited to actinomycosis, anthrax, bacterial dysentery, botulism, brucellosis, cellulitis, cholera, conjunctivitis, cystitis, diphtheria, bacterial endocarditis, epiglottitis, gastroenteritis, glanders, gonorrhea, Legionnaire's disease, leptospirosis, bacterial meningitis, plague, bacterial pneumonia, puerperal sepsis, rheumatic fever, Rocky Mountain spotted fever, scarlet fever, streptococcal pharyngitis, syphilis, tetanus, tularemia, typhoid fever, typhus, and pertussis.

The following Examples are provided for the purposes of illustration and are not intended to limit the scope of the present invention. The present invention is not to be limited in scope by the exemplified embodiments, which are intended as illustrations of individual aspects of the invention. Indeed, various modifications of the invention in addition to those shown and described herein will become apparent to those skilled in the art from the foregoing description and accompanying drawings. Such modifications are intended to fall within the scope of the appended claims.

### EXAMPLE 1

### Compounds based on Diphenyl Borinic Esters

Several compounds based on diphenyl borinic ester were prepared as follows. The general synthesis of these compounds is shown in Reaction Scheme 1. 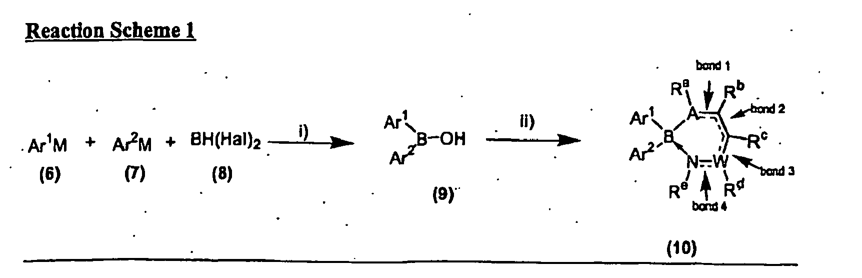 where the reaction conditions are:
i) tetrahydrofuran (THF) or ethyl ether (Et₂O), -78°C to room temperature overnight;
ii) EtOH, room temperature, boron coordinating agent;
and where M = MgBr, Li
Hal = Cl, Br
A = O, N, S
W = Cₚ where p = 0,1
R^{a}, R^{b}, R^{c}, R^{d}, and R^{e} are the same or different and are independently hydrogen, halogen, nitro, nitroso, lower alkyl, aryl or substituted aryl, lower alkoxy, lower alkoxyalkyl, or cycloalkyl or cycloalkyl alkoxy, where each cycloalkyl group has from 3-7 members, where up to two of the cycloalkyl members are optionally hetero atoms selected from sulfur, oxygen and nitrogen, and where any member of the alkyl, aryl or cycloalkyl group is optionally substituted with halogen, lower alkyl or lower alkoxy, aryl or substituted aryl, halogen, nitro, nitroso, aldehyde, carboxylic acid, amide, ester, or sulfate, or wherein R^{a}, R^{b}, R^{c}, R^{d}, and R^{e} may be connected by aromatic, aliphatic, heteroaromatic, heteroaliphatic ring structures or substituted embodiments thereof; where R^{a} is absent when A is O or S and R^{d} is absent when p = 0
bond 2, bond 3 and bond 4 are independently a single or a double bond, and when A = O, S, bond 1 is a single bond and when A = N, bond 1 is a single or a double bond
and
X can represent up to 5 substituents on each phenyl group, which can be independently hydrogen, lower alkyl, aryl or substituted aryl, lower alkoxy, lower alkoxyalkyl, or cycloalkyl or cycloalkyl alkoxy, where each cycloalkyl group has from 3-7 members, where up to two of the cycloalkyl members are optionally hetero atoms selected from oxygen and nitrogen, and where any member of the alkyl, aryl or cycloalkyl group is optionally substituted with halogen, lower alkyl or lower alkoxy, aryl or substituted aryl, halide, nitro, nitroso, aldehyde, carboxylic acid, esters, amides, or sulfates.

Preferred compounds are identified herein based on the identity of the substituents Ar¹ = Ar² where: 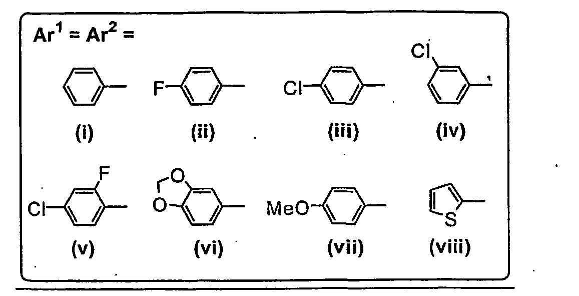 are in combination with any one of the following:

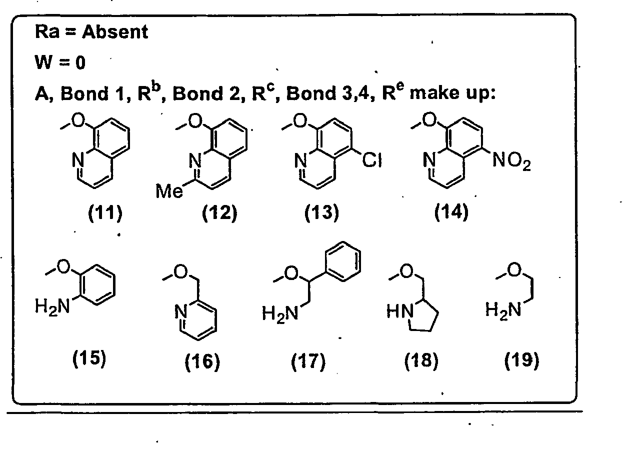

### General Experimental Protocols

Chemicals were purchased from Acros Organics and Aldrich Chemical Company (Milwaukee, WI) and were used without further purification. Tetrahydrofuran was dried by distillation from sodium and benzophenone; diethyl ether was dried over sodium and distilled; all other solvents used were the highest available grade and used without further purification.

Reactions were performed as set forth in detail below. Reaction products were analyzed by ¹H-NMR spectra recorded on a Bruker Avance 400 (400 MHz) and Bruker AMX360 (360 MHz). MALDI mass spectra were obtained using a Perspective Biosystems Voyager-DE STR, FAB mass spectra were obtained using a Kratos Analytical MS-50 TC, and APCI mass spectra were recorded on a Perspective Biosystems Mariner. Microanalyses were recorded by Atlantic Microlab Inc. (Norcross, Georgia 30091). Analytical thin layer chromatography (tlc) was performed with Whatman silica gel aluminum backed plates of thickness 250 µm and fluorescent at 254 nm, and by using the solvent systems indicated. Flash column chromatography was performed with Selecto Scientific silica gel, 32-64 µm particle size. Melting points were obtained using a Mel-Temp II melting point apparatus with a Fluke K1 K/J type thermocouple digital thermometer and are uncorrected. Purity was determined by HPLC using a betabasic-18 (4.6 mm x 15 cm) column from Keystone Scientific Inc. and product eluted using a linear gradient of 0 to 40 % acetonitrile in 10 mM triethyl ammonium acetate over 20 mins.

*Caulobacter crescentus* strain CB15N was a gift from Prof. Lucille Shapiro, Department of Developmental Biology, Stanford University, Stanford, CA 94305; USA. *Bacillus subtilis* (ATCC #33234) was obtained from ATCC, Manassas, VA.

### General methods for the synthesis of diaryl borinic acids (9)

**Method A:** Dichloroborane dimethyl sulfide complex or dibromoborane dimethyl sulfide (1 molar equivalent) was added to either tetrahydrofuran (0.2 mmol/mL) or diethyl ether (0.2 mmol/mL) under argon and cooled to -78°C. The aryl magnesium bromide (2 molar equivalents), in tetrahydrofuran, diethyl ether, cyclohexane, toluene or mixtures of these solvents, was added dropwise to the cold reaction mixture. The reaction was allowed to warm to room temperature and stirred overnight. The solvents were removed *in vacuo* and the residue was dissolved in diethyl ether. The reaction was stirred rapidly and hydrolyzed by the slow addition of 1N hydrochloric acid. Stirring was discontinued, the layers were separated and the organic layer was washed with saturated aqueous NaCl. The organic layer was dried over magnesium sulfate (MgSO₄), filtered and the solvent removed *in vacuo* to give the crude product as an oil. This oil was dissolved in ethanol to an estimated concentration of 1M and divided into portions to be used in the subsequent precipitation with the various complexing agents.
**Method B:** Dichloroborane dimethyl sulfide complex or dibromoborane dimethyl sulfide complex (1 molar equivalent) was added to either tetrahydrofuran (2 mmol/mL) or diethyl ether (2 mmol/mL) under argon and cooled to -78°C. The aryl lithium (2 molar equivalents), in tetrahydrofuran, diethyl ether, cyclohexane or mixtures of these solvents, was added dropwise to the cold reaction mixture. The reaction was allowed to warm to room temperature and stirred overnight. The solvents were removed *in vacuo* and the residue was dissolved in diethyl ether. The reaction mixture was stirred rapidly and hydrolyzed by the slow addition of 1N hydrochloric acid. Stirring was discontinued, the layers were separated and the organic layer was washed with saturated aqueous NaCl. The organic layer was dried over magnesium sulfate (MgSO₄), filtered and the solvent removed *in vacuo* to give the crude product as an oil. This, oil was dissolved in ethanol to an estimated concentration of 1M and divided into portions to be used in the subsequent precipitation with the various complexing agents.

Products are identified herein as a combination of the constituents identified above. Thus, di-(4-fluorophenyl)borinic acid 8-hydroxyquinoline ester is identified as 11ii, indicating that Ar¹ = Ar² and are each 4-fluorophenyl (substituent ii above), and R^{a} is absent, p = 0, and A, bond 1, R^{b}, bond 2, R^{c}, bond 3, bond 4 and R^{e} make up hydroxyquinoline (substituent 11 above).

**Di-(4-fluorophenyl)borinic acid 8-hydroxyquinoline ester (11ii),** Di-(4-fluorophenyl)borinic acid was prepared using method A and was treated with 8-hydroxyquinoline (0.5M solution in ethanol). A yellow precipitate formed immediately and was collected by filtration and washed with ethanol. The product had the following properties upon analysis: mp 166-167°C; ¹H-NMR (360 MHz, C²HCl₃): δ 8.53 (d, *J* = 5.1 Hz, 1H), 8.45 (d, *J* = 8.2 Hz, 1H), 7.70 (dd, *J* = 8.2, 7.7 Hz, 1H), 7.66 (dd, *J=* 8.2, 5.1 Hz, 1H), 7.39 (dd, *J =* 8.8, 6.7 Hz, 4H), 7.30 (d, *J=* 8.2 Hz, 1H), 7.20 (d, *J=* 7.7 Hz, 1H), 6.97 (t, *J*= 8.8 Hz, 4H); MS (+ve APCI) m/z 345 ([M+H]⁺, ¹⁰B), 346 ([M+H]⁺, ¹¹B), 368 ([M+Na]⁺, ¹¹B); Anal. (C₂₁H₁₄NOBF₂) C, H, N.

**Di-(4-chlorophenyl)borinic acid 8-hydroxyquinoline ester (11iii):** Di-(4-chlorophenylborinic acid was prepared using method A and was treated with 8-hydroxyquinoline (0.5M solution in ethanol). A yellow precipitate formed immediately and was collected by filtration and washed with ethanol. The product had the following properties upon analysis: mp 192-194 °C; ¹H-NMR (360 MHz, C²HCl₃,): δ 8.49 (dd, *J=* 4.6, 1.0 Hz, 1H), 8.43 (dd, *J* = 8.2,1.0 Hz, 1H), 7.70 (dd, *J =* 8.5, 7.7Hz, 1H), 7.66 (dd, *J*= 8.2, 4.6 Hz, 1H), 7.31 (d, *J=* 8.2 Hz, 4H), 7.26 (d, *J=* 8.5 Hz, 1H), 7.21 (d, *J=* 8.2 Hz, 4H), 7.17 (d, *J=* 7.7 Hz, 1H); MS (+ve APCI) m/z 377 ([M+H]⁺, ¹⁰B, ³⁵Cl, ³⁵Cl), 378 ([M+H]⁺, ¹¹B, ³⁵Cl, ³⁵Cl), 379 ([M+H]⁺, ¹⁰B, ³⁵Cl, ³⁷Cl), 380 ([M+H]⁺, ¹¹B, ³⁵Cl, ³⁷Cl), 381 ([M+H]⁺, ¹⁰B, ³⁷Cl, ³⁷Cl), 382 ([M+H]⁺, ¹¹B, ³⁷Cl, ³⁷Cl); Anal. (C₂₁H₁₄NOBCl₂) C, H, N.

**Di-(3-chlorophenyl)borinic acid 8-hydroxyquinoline ester (11iv): Di-(3-**chlorophenyl)borinic acid was formed using method A and was treated with 8-hydroxyquinoline (1.0 M in ethanol). The solvent was removed *in vacuo* and the residue was purified by flash chromatography on silica gel using CH₂Cl₂/hexane (1:1) to elute the product. The solvent was evaporated and the product crystallized upon the addition of ethanol. The solid was collected by filtration and washed with cold ethanol to yield the title product as a yellow solid having the following properties: mp 144-145°C ; ¹H-NMR (360 MHz, C²H₃O²H): 8.83 (d, *J =* 5.0 Hz, 1H), 8.63 (d, *J* = 8.2 Hz, 1H), 7.78 (dd, *J* = 8.6, 5.0 Hz, 1H), 7.67 (t, *J* = 8.2, 1H), 7.36 (d, *J* = 8.2, 1H), 7.26-7.09 (m, 9H); MS (+ve ESI) 377 ([M+H]⁺, ¹⁰B, ³⁵Cl, ³⁵Cl), 378 ([M+H]⁺, ¹¹B, ³⁵Cl, ³⁵Cl), 379 ([M+H]⁺, ¹⁰B, ³⁵Cl, ³⁷Cl), 380 ([M+H]⁺, ¹¹B, ³⁵Cl, ³⁷Cl), 381 ([M+H]⁺, ¹⁰B, ³⁷Cl, ³⁷Cl), 382 ([M+H]⁺, ¹¹B, ³⁷Cl, ³⁷Cl); Anal. (C₂₁H₁₄NOBCl₂) C, H, N.

**Di-(4-chloro-2-fluorophenyl)borinic acid 8-hydroxyquinoline ester (11v):** Di-(4-chloro-2-fluorophenyl)borinic acid was formed using method A and was treated with 8-hydroxyquinoline (0.5M in ethanol). The residue was purified by flash chromatography on silica gel using hexane/ethyl acetate (3:1). The solvent was evaporated and the product crystallized upon the addition of ethanol. The solid was collected by filtration and washed with cold ethanol to yield the title product as a yellow solid having the following properties: mp 135°C; ¹H-NMR (360 MHz, C²H₃O²H): 8.82 (d, *J*= 5.0 Hz, 1H), 8.61 (d, *J*= 8.2 Hz, 1H), 7.75 (dd, *J*= 8.2, 5.0 Hz, 1H), 7.63 (t, *J*= 8.2 Hz, 1H), 7.35 (d, *J=* 8.2 Hz, 1H), 7.27 (t, *J=* 7.7 Hz, 2H), 7.13-6.90 (m, 5H); MS (+ve APCI) 413 ([M+H]⁺, ¹⁰B, ³⁵Cl, ³⁵Cl), 414 ([M+H]⁺, ¹¹B, ³⁵Cl, ³⁵Cl), 415 ([M+H]⁺, ¹⁰B, ³⁵Cl, ³⁷Cl), 416 ([M+H]⁺, ¹¹B, ³⁵Cl, ³⁷Cl), 417 ([M+H]⁺, ¹⁰B, ³⁷Cl, ³⁷Cl), 418 ([M+H]⁺, ¹¹B, ³⁷Cl, ³⁷Cl); Anal. calcd for C₂₁H₁₂NOBF₂Cl₂(0.5 H₂O): C 59.62, H 3.10, N 3.31; found: C 59:74, H 3.03, N 3.18.

**Di-(3,4-methylenedioxyphenyl)borinic acid 8-hydroxyquinoline ester (11vi):** Di(3,4-methylenedioxyphenyl)borinic acid was formed using method A and was treated with 8-hydroxyquinoline (0.5M in ethanol). The solution was allowed to stand overnight to crystallize. The solid was collected by filtration and washed with cold ethanol to yield the product as a yellow solid having the following properties: mp 174-176 °C; ¹H-NMR (400 MHz, C²HCl₃): 8.52 (d, *J =* 4.9 Hz, 1H), 8.41 (d, *J =* 8.2 Hz, 1H), 7.66 (t, *J* = 7.9 Hz, 1H), 7.63 (dd, *J =* 8.2, 5.0 Hz, 1H), 7.25 (d, 1H), 7.17 (d, *J=* 7.7 Hz, 1H), 6.91 (s, 2H), 6.89 (d, *J=* 8.5 Hz, 2H), 6.76 (d, *J=* 8.2 Hz, 2H), 5.87 (s, 4H); MS (+ve APCI) m/z 397 (M⁺,¹⁰B), 398 (M⁺, ¹¹B); Anal. (C₂₃H₁₆BNO₅) C, H, N.

**Di-(4-methoxyphenyl)borinic acid 8-hydroxyquinoline ester (11vii): Di(4-**methoxyphenyl) borinic acid was formed using method A and was treated with 8-hydroxyquinoline (0.5M in ethanol) causing the title product to precipitate from the solution. The solid was collected by filtration and washed with cold ethanol to yield the product as a yellow solid having the following properties: mp 222-224°C; ¹H-NMR (400 MHz, C²HCl₃): 8.53 (d, *J* = 5.0 Hz, 1H), 8.38 (d, *J* = 8.5 Hz, 1H), 7.67 (t, *J =* 8.0 Hz, 1H), 7.61 (dd, *J =* 8.3, 5.0 Hz, 1H), 7.38 (d, *J* = 8.5 Hz, 4H), 7.24 (d, *J* = 8.2 Hz, 1H), 7.17 (d, *J* = 7.7 Hz, 1H), 6.85 (d, *J* = 8.6 Hz, 4H), 3.78 (s, 6H); MS (+ve MALDI, CHCA) m/z 369 ([M+H]⁺, ¹⁰B), 370 ([M+H]⁺, ¹¹B); Anal. (C₂₃H₂₀BNO₃) C, H, N.

**Di-(2-thienyl)borinic acid 8-hydroxyquinoline ester (11viii): Di-(2-**thienyl)phenylborinic acid was prepared using method 2 and was treated with 8-hydroxyquinoline (0.5M solution in ethanol). A yellow precipitate formed upon standing and was collected by filtration and washed with ethanol. The isolated product had the following properties: mp 151-152 °C; ¹H-NMR (360 MHz, C²HCl₃): δ 8.58 (d, 1H, *J*= 5.4 Hz), 8.39 (d, *J* = 8.7 Hz, 1H), 7.62 (t, *J*=.8.2, 1H), 7.60 (dd, *J =* 8.7, 5.4 Hz, 1H), 7.39 (dd, *J =* 4.6, 1.0 Hz, 4H), 7.26 (d, *J =* 8.2 Hz, 1H), 7.22 (dd, *J =* 3.4, 1.0 Hz, 2H), 7.20 (d, *J =* 8.2 Hz, 1H), 7.08 (dd, *J =* 4.6, 3.4 Hz, 2H); MS (+ve APCI) m/z 321 ([M+H]⁺, ¹⁰B), 322 ([M+H]⁺, ¹¹B); Anal. Calc for C₁₇H₁₂ BONS₂ 0.7(H₂O): C 61.17, H 4.05, N 4.20; found C 61.15, H 4.09, N 4.25.

**Di-(p-fluorophenyl)borinic acid 8-hydroxyquinaldine ester (12ii): Di-(p-**fluorophenyl) borinic acid was formed using method A and was treated with 8-hydroxyquinaldine (0.5M solution in ethanol). The product was collected by filtration and washed with ethanol. The purified product had the following properties: mp 154-156 °C; ¹H-NMR (360 MHz, C²HCl₃): 8.21 (d, *J =* 8:7 Hz, 1H) 7.47 (t, *J =* 8.2 Hz, 1H), 7.28 (d, *J*= 8.4, 1H), 7.22 (m, 4H), 7.12 (d, *J =* 8.1 Hz, 1H), 6.98 (d, *J*= 7.8 Hz, 1H), 6.85 (m, 4H) 2.39 (s, 3H); MS (+ve, APCI) m/z 359 ([M+H]⁺, ¹⁰B), 360 ([M+H]⁺, ¹¹B); Anal. (C₂₂H₁₆NBOF₂) C, H, N.

**Di-(p-chlorophenyl)borinic acid 8-hydroxyquinaldine ester (12iii): Di-(p-**chlorophenyl)-borinic acid was formed using method A and was treated with chloroquinaldine (0.5M solution in ethanol). The product was collected by filtration and washed with ethanol. The purified product had the following properties: mp 155-156 °C; ¹H-NMR (360 MHz, C²HCl₃): 8.21 (d, *J* = 8.6 Hz, 1H), 7.46 (t, *J*= 8.4 Hz, 1H), 7.27 (d, *J* = 8.2 Hz, 1H), 7.18 - 7.11 (m, 9H), 6.97 (d, *J* = 7.8 Hz, 1H) 2.38 (s, 3H); MS (+ve, APCI) m/z 392 (M⁺, ¹¹B, ³⁵Cl, ³⁵Cl), 394 (M⁺, ¹¹B, ³⁵Cl, ³⁷Cl), 396 (M⁺, ¹¹B, ³⁷Cl, ³⁷Cl); Anal. (C₂₂H₁₆NBOCl₂) C, H, N.

**Di-(4-methoxyphenyl)borinic acid 8-hydroxyquinaldine ester (12vii):** Di-(4-methoxyphenyl) borinic acid was formed using method A and was treated with 8-hydroxyquinaldine (0.5M in ethanol). The solution was allowed to stand overnight to crystallize. The solid was collected by filtration and washed with cold ethanol to yield the title product as a yellow solid having the following properties: mp 150-151 °C; ¹H-NMR (400 MHz, C²HCl₃): 8.29 (d, *J* = 8.4 Hz, 1H), 7.57 (t, *J* = 8.0 Hz, 1H), 7.37 (d, *J =* 8.4 Hz, 1H), 7.33 (d, *J*= 8.5, 4H), 7.20 (d, *J*= 8.2 Hz, 1H), 7.08 (d, *J =* 7.7 Hz, 1H), 6.84 (d, *J* = 8.5 Hz, 4H), 3.79 (s, 6H), 2.54 (s, 3H); MS (+ve MALDI, CHCA) m/z 383 ([M+H]⁺, ¹⁰B), 384 ([M+H]⁺, ¹¹B); Anal. (C₂₄H₂₂BNO₃) C, H, N.

**Di-(p-fluorophenyl)borinic acid 5-chloro-8-hydroxyquinaline ester (13ii): Di-**(p-fluorophenyl) borinic acid was formed using method A and was treated with 5-chloro-8-hydroxy quinaline (0.5M solution in ethanol). The product was collected by filtration and washed with ethanol. The purified product had the following properties: mp 143-145 °C; ¹H-NMR (360 MHz, C²HCl₃): 8.55 (d, *J* = 8.3 Hz, 1H), 8.45 (d, *J* = 5.0 Hz, 1H), 7.63 (m, 1H), 7.58 (d, *J* = 8.15 Hz, 1H), 7.22 (m, 4H), 6.98 (d, *J*= 8.14 Hz, 1H), 6.84 (m, 4H); MS (+ve, APCI) m/z 380 ([M+H]⁺, ¹¹B); Anal. (C₂₁H₁₃NBOClF₂) C, H, N.

**Di-(p-chlorophenyl)borinic acid 5-chloro-8-hydroxyquinaline ester (13iii):** Di-(p-chlorophenyl)borinic acid was formed using method A and was treated with 5-chloro-8-hydroxy quinaline (0.5M solution in ethanol). The product was collected by filtration and washed with ethanol. The purified product had the following properties: mp = 154-156°C; ¹H-NMR (360 MHz, C²HCl₃): 8.56(d, *J*= Hz, 1H), 8.44 (d, *J* = Hz, 1H), 7.64 (m, 1H), 7.57 (d, *J* = Hz, 1H), 7.14 (m, 9 H), 6.98 (d, 1H); MS (+ve, ESI) m/z 412 ([M+H]⁺, ¹⁰B, ³⁵Cl, ³⁵Cl, ³⁵Cl), 413 ([M+H]⁺, ¹¹B, ³⁵Cl, ³⁵Cl, ³⁵Cl); Anal. (C₂₁H₁₃NBOCl₂) C, H, N.

**Di-(3,4-methylenedioxyphenynl)borinic acid 5-chloro-8-hydroxyquinoline ester (13vi):** Di-(3,4-methylenedioxyphenyl)borinic acid was formed using method A and was treated with hot 5-chloro-8-hydroxyquinoline (0.5M in ethanol). The solution was allowed to stand overnight to crystallize. The solid was collected by filtration and washed with cold ethanol to yield the product as a yellow solid having the following properties: mp 212-213°C; ¹H-NMR (360 MHz, C²HCl₃): 8.66 (d, *J =* 8.4 Hz, 1H), 8.58 (d, *J =* 4.9 Hz, 1H), 7.75 (dd, *J =* 8.5, 5.1 Hz, 1H), 7.69 (d, *J =* 7.3 Hz, 1H), 7.09 (d, *J =* 8.3 Hz, 1H), 6.88 (s and d, overlapping, 4H), 6.76 (d, *J* = 7.6 Hz, 2H), 5.88 (s, 4H); MS (+ve, APCI) m/z 432 ([M+H]⁺, ¹¹B, ³⁵Cl), 434 ([M+H]⁺, ¹¹B, ³⁷Cl); Anal. (C₂₃H₁₅BClNO₅) C, H, N.

**Di-(4-methoxyphenyl)borinic acid 5-chloro-8-hydroxyquinoline ester (13vii):** Di-(4-methoxyphenyl)borinic acid was formed using method A and was treated with hot 5-chloro-8-hydroxyquinoline (0.5m in ethanol). The solution was allowed to stand overnight to crystallize. The solid was collected by filtration and washed with cold ethanol to yield the title product as a yellow solid having the following properties: mp 184-185°C; ¹H-NMR (400 MHz, C²HCl₃): 8.66 (d, *J* = 8.3 Hz, 1H), 8.59 (d, *J* = 5.1 Hz, 1H), 7.73 i(dd, *J =* 8.4, 5.1 Hz, 1H), 7.69 (d, *J* = 8.3 Hz, 1H), 7.34 (d, *J* = 8.6 Hz, 4H), 7.09 (d, *J =* 8.3 Hz, 1H), 6.84 (s, *J =* 8.6 Hz, 4H), 3.78 (s, 6H); MS (+ve APCI) m/z 403 (M⁺, ¹¹B, ³⁵Cl), 405 (M⁺, ¹¹B, ³⁷Cl); Anal. (C₂₃H₁₉BClNO₃) C, H, N.

**Di-(3,4-methylenedioxyphenyl)borinic acid 8-hydroxy-5-nitroquinoline ester (14vi):**
Di-(3,4-methylenedioxyphenyl)borinic acid was formed using method A and was treated with hot 8-hydroxy-5-nitroquinoline (0.5m in ethanol). The solution was allowed to stand overnight to crystallize. The solid was collected by filtration and washed with cold ethanol to yield the product as a. yellow solid having the following properties: mp 243-245°C; ¹H-NMR (360 MHz, C²HCl₃): 9.69 (d, *J* = 8.7 Hz, 1H), 8.90 (d, *J=* 8.8 Hz, 1H), 8.68 (d, *J=* 5.0 Hz, 1H), 7.97 (dd, *J=* 8.7, 5.0 Hz, 1H), 7.17 (d, *J =* 8.9 Hz, 1H), 6.84 (s, 2H), 6.82 (d, *J =* 7.8 Hz, 2H), 6.77 (d, *J* = 7.7 Hz, 2H), 5.90 (s, 4H); MS (+ve APCI) 442 (M⁺, ¹¹B); Anal. (C₂₃H₁₅BN₂O₇) C, H, N.

**Diphenylborinic acid 2-aminophenol (15i):** Diphenylborinic acid was prepared using method B and was treated with 2-aminophenol (1M solution in ethanol). A white precipitate formed upon standing and was collected by filtration and washed with ethanol. The purified product had the following properties: mp 179-181 °C; ¹H-NMR (360 MHz, C²H₃O²H): δ 7.44 (m, 4H), 7.18 (m, 6H), 6.90 (dd, 1H), 6.76 (dd, 1H), 6.62 (m, 2H); MS (+ve,APCI) m/z 274 ([M+H]⁺, ¹¹B); Anal. (C₁₈H₁₆NOB): C, H, N.

**Diphenylborinic acid pyridine-2-methanol (16i):** Diphenylborinic acid was prepared using method B and was treated with pyridine-2-methanol (1M solution in ethanol). A white precipitate formed upon standing and was collected by filtration and washed with ethanol; mp 152-153°C; ¹H-NMR (360 MHz, C²HCl₃): δ 8.40 (d, *J=* 6.0 Hz, 1H), 7.98 (t, *J=* 7.7 Hz, 1H), 7.54 (d, *J*= 7.7 Hz, 1H), 7.30 (m, 4H), 7.23 (m, 7H); MS (+ve, APCI) m/z 274 ([M+H]⁺, ¹¹B); Anal. (C₁₈H₁₆NOB): C, H, N.

**Diphenylborinic acid 2-amino-1-phenylpropanol (17i):** Diphenylborinic acid was prepared using method B and was treated with 2-amino-1-phenylpropanol (1M solution in ethanol). A white precipitate formed upon standing and was collected by filtration and washed with ethanol. The purified product had the following properties: mp 200-201°C; ¹H-NMR (360 MHz, C²H₃O²H): δ 7.70-7.10 (m, 15H), 5.03 (dd, *J* = 9.2, 6.4 Hz, 1H), 3.32 (dd, *J*= 10.9, 6.4 Hz, 1H), 2.81 (dd, *J=* 10.9, 9.2 Hz, 1H); MS (+ve, APCI) m/z 302 ([M+H]⁺, ¹¹B); Anal. (C₂₀H₂₀NOB) C, H, N.

**Diphenylborinic acid (S)-(+)-pyrrolidine-2-methanol (18i):** Diphenylborinic acid was prepared using method B and was treated with (S)-(+)-pyrrolidine-2-methanol (1M solution in ethanol). A white precipitate formed upon standing and was collected by filtration and washed with ethanol. The purified product had the following properties: mp 221-222°C; ¹H-NMR (360 MHz, C²H₃O²H): δ 7.44 (m, 4H), 7.13 (m, 6H), 3.94 (dd, *J=* 6.6, 8.7 Hz, 1H), 3.77 (m, 1H), 3.68 (dd, *J*= 6.6, 8.7 Hz, 1H), 2.86 (m, 1H), 2.60 (m, 1H), 2.14 (m, 1H), 1.93-1.65 (m, 3H); MS (+ve, APCI) m/z 266 ([M+H]⁺, ¹¹B); Anal. (C₁₇H₂₀NOB) C, H, N.

**Di-(4-fluorophenyl)borinic acid ethanolamine ester (19ii):** Di-(4-fluorophenyl)borinic acid was prepared using method A and was treated with ethanolamine (1M solution in ethanol). A white precipitate formed upon standing and was collected by filtration and washed with ethanol. The purified product had the following properties: mp 247-249°C;¹H-NMR (360 MHz, C²H₃O²H): δ 7.37 (dd, *J=* 8.7, 6.4 Hz, 4H), 6.90 (t, *J*= 8.7 Hz, 4H), 3.95 (d, *J=* 6.4 Hz, 2H), 3.02 (d, *J=* 6.4 Hz, 2H); MS (+ve APCI) m/z 261 ([M+H]⁺, ¹⁰B), 262 ([M+H]⁺, ¹¹B); Anal. (C₁₄H₁₄NOBF₂) C, H, N,

**Di-(4-chlorophenyl)borinic acid ethanolamine ester (19iii):** Di-(4-chlorophenylborinic acid was prepared using method A and was treated with ethanolamine (1M solution in ethanol). A white precipitate formed upon standing and was collected by filtration and washed with ethanol. The purified product had the following properties: mp 241-242°C; ¹H-NMR (360MHz, C²H₃O²H): δ 7.35 (d, *J* = 8.6, 4H), 7.18 (d, *J =* 8.6, 4H), 3.94 (d, *J =* 6.4 Hz, 2H), 3.02 (d, *J* = 6.4 Hz, 2H); MS (+ve APCI) m/z 293 ([M+H]⁺, ¹⁰B, ³⁵Cl, ³⁵Cl), 294 ([M+H]⁺, ¹¹B, ³⁵Cl, ³⁵Cl), 295 ([M+H]⁺, ¹⁰B, ³⁵Cl, ³⁷Cl), 296 ([M+H]⁺, ¹¹B, ³⁵Cl, ³⁷Cl), 297 ([M+H]⁺, ¹⁰B, ³⁷Cl, ³⁷Cl), 298 ([M+H]⁺, ¹¹B, ³⁷Cl, ³⁷Cl); Anal. (C₁₄H₁₄NOBCl₂) C, H, N.

### EXAMPLE 2

### Biological Activity Assays

The antibacterial activity of the compounds prepared as descried above were tested using *Caulobacter crescentus* and *Bacillus subtilis* as follows.

### Caulobacter crescentus cell growth assay

*Caulobacter crescentus* (CB15N) was grown in PYE media (Sambrook *et al.,* 1989, Molecular Cloning: A Laboratory Manual, 2d ed., Cold Spring Harbor Press: N.Y.) overnight at 30°C to saturation. Taking advantage of the inherent ampicillin resistance of *Caulobacter crescentus* to minimize the risk of contamination, this saturated culture was diluted in PYE media containing 200 µg/mL ampicillin to a final OD₆₀₀ of 0.05. Aliquots (146 µL) of this diluted cell culture were placed in wells of a microtiter plate. An inhibitor (4 µL of a stock solution of an appropriate concentration dissolved in either dimethylformamide or dimethylsulfoxide) was added to each of these wells to give a final volume of 150 µL. This plate was incubated at 30°C with gentle shaking at 550 rpm in an Eppendorf Thermomixer R. Control experiments were performed in parallel. These consisted of wells containing: i) 150 µL PYE/ampicillin media (no cell culture) as a blank; and ii) 146 µL diluted cell culture and 4 µL (DMF or DMSO) for maximum cell growth in the presence of solvent and absence of inhibitor. Cell growth was monitored at 630 nm using a microtitre plate reader at time points of: 0 hours, 4 hours, 8 hours, and 22 hours. The final cell growth was recorded as a percent of the maximum cell growth.

### Bacillus subtilis cell growth assay

A similar cell growth assay to that described for *Caulobacter crescentus* was performed with *Bacillus subtilis* (ATCC #33234). The following changes were incorporated to accommodate the different growth conditions: i) cells were grown in Luria-Bertani (LB) media without antibiotics (Sambrook *et al., ibid.);* ii) growth temperature was 37°C; and iii) cell growth was monitored at time points of 0 hours, 2.5 hours, 5 hours, and 7.5 hours. After 7.5 hours any culture with inhibited cell growth was diluted 500-fold into fresh LB media. Recovery from inhibitor selection was assessed after 12 hours at 37°C.

### CcrM inhibition assay

Methyltransferase activity was measured by monitoring the incorporation of [³H]CH₃ from [³H]-*S*-adenosylmethionine (SAM) into DNA. A stock solution containing 250 nM CcrM, 5 µM N645/50 mer (the sequence of which is identified below), 150 mM potassium acetate, 5 mM 2-mercaptoethanol in pH 7.5 HEPES buffer was prepared. Aliquots were placed in Eppendorf tubes and inhibitors were added from concentrated stock solutions (16.7 mM in DMF or DMSO) to reach the appropriate final concentrations (500 µM or 100 µM) in a total of 15 µL. Reactions were initiated by the addition of [³H]SAM at a final concentration of 50 µM. After 40 mins at 30°C, 5 µL aliquots were removed from the reaction and spotted onto DE81 anion exchange filter circles. The filters were allowed to dry and then washed with 3 x 200 mL of 0.3 M ammonium formate to remove unreacted [³H]SAM, followed by 2 x 200 mL 95 % ethanol wash and finally a 200 mL ether wash. The filters were allowed to air dry and counted by standard liquid scintillation techniques. Control reactions in, the absence of inhibitors were used to determine the extent of inhibition. High throughput screening was carried out similarly in Tris-HCl buffer (50 µM, pH 7.5) with plasmid DNA as substrate (Litmus 29 (New England Biolabs): 250 µM DNA, 3 µM sites), 100 µM inhibitor candidate and enzyme, potassium acetate and 2-mercaptoethanol concentrations as described above. Assays were initiated with [¹⁴CH₃]SAM (50 µM, 34 Ci/mol) in a volume of 10 µL in a PCR plate, and incubated for 30 minutes at 30°C. Four microliter aliquots were then spotted on DE81 paper with a multichannel pipette and washed and dried as described above. Data was collected with a Molecular Dynamics model 425S phosphorimager, and analyzed with the spotfinder utility in ImageQuant 3.3.

### DNA substrate [N645/50 mer]

CH3
5'-ATC CTC TCG CGA ATC AAC AGA AAT ATC CGC TCA TCA CCG CAA GTT
3'- AG GAG AGC GCT TAG TTG TCT TTA TAG GCG AGT AGT GGC GTT CAA AAG GCA A

(where the methylated strand shown above is SEQ ID No. 1. and the complementary strand is SEQ ID No. 2). Synthesis of DNA was achieved on an Expedite BioSystems DNA synthesizer and purified as previously described (Capson *et al.,* 1992, *Biochemistry* 31: 10984-10994).

**Table I**

| | % reaction | IC₅₀ (µM) | |
|---|---|---|---|
| Compound | *Brucella abortus* CcrM Activity | *Caulobacter crescentus* Cell Growth | *Bacillus subtilis* Cell Growth |
| **(11i)** | | >100 | nt |
| **(11ii)** | 61 | 23 | nt |
| **(11iii)** | 61 | 16 | nt |
| **(11iv)** | | 10 | <10 |
| **(11v)** | | 26 | <10 |
| **(11vi)** | 42 | 32 | 28 |
| **(11vii)** | | 24 | 20 |
| **(11viii)** | 74 | >100 | 28 |
| **(12ii)** | 17 | 56 | >100 |
| **(12iii)** | 40 | 5 | 24 |
| **(12vii)** | 56 | 24 | >100 |
| **(13ii)** | 55 | 7 | 27 |
| **(13iii)** | 81 | 6 | <10 |
| **(13vi)** | | 7 | >100 |
| **(13vii)** | | 7 | 36 |
| **(14vi)** | 21 | 7 | >100 |
| **(15i)** | 38 | >100 | >100 |
| **(16i)** | 93 | >100 | >100 |
| **(17i)** | 114 | >100 | >100 |
| **(18i)** | 81 | >100 | >100 |
| **(19ii)** | 8 | 85 | nt |
| **(19iii)** | | 17 | nt |

These results indicate that the compounds of the invention have useful IC50 values for inhibiting CcrM and DAM methylases in bacteria.

These compounds have advantageous physical properties, and are isolated as pure, stable solids that are amenable to large-scale production. Additional specific embodiments of adenine DNA methyltransferase inhibitors of the invention includes related compounds having these additional features:
1) Analogues with various substituents on the phenyl rings in any, or combination of, the ortho-, meta- and para- positions, including fused rings and substituted fused rings;
2) Analogues having aromatic heterocycles of various ring sizes, substituted heterocycles, fused heterocycles and substituted fused heterocycles in place of one or both phenyl groups;
3) Analogues having two non-identical aromatic rings bound to the boron atom, using combinations of the aromatic systems described in 1) and 2) above;
4) Analogues prepared using quinolines **(9)** containing various substituents in any possible position or structural analogues including fused heteroaromatic rings containing one or more heteroatom in any possible position or fused heteroaromatic rings containing one or more heteroatom in any possible position and containing various substituents in any possible position; and
5) Analogues having substitutions on either, or both of, the C-1 and C-2 positions of the ethylene group of the 2-aminoethanol of **(10).**

## Claims

1. A pharmaceutical composition for use in humans, comprising a compound of the formula: 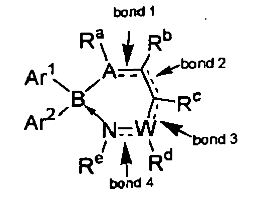 including all pharmaceutically acceptable salts of such compounds, and at least one pharmaceutically acceptable carrier or excipient;
wherein A is N, O or S;
W is Cₚ, where p is 0 or 1;
R^{a}, R^{b}, R^{c}, R^{d}, and R^{e} are the same or different and are independently hydrogen, halogen, lower alkyl, aryl or substituted aryl, lower alkoxy, lower alkoxyalkyl, or cycloalkyl or cycloalkyl alkoxy, where each cycloalkyl group has from 3-7 members, where up to two of the cycloalkyl members are optionally hetero atoms selected from sulfur, oxygen and nitrogen, and where any member of the alkyl, aryl or cycloalkyl group is optionally substituted with halogen, lower alkyl or lower alkoxy, aryl or substituted aryl, halogen, nitro, nitroso, aldehyde, carboxylic acid, amide, ester, or sulfate, or wherein R^{a}, R^{b}, R^{c}, R^{d}, and R^{e} may be connected by aromatic, aliphatic, heteroaromatic, heteroaliphatic ring structures or substituted embodiments thereof, where R^{a} is absent when A is O or S and R^{d} is absent when p = 0; and
wherein Ar¹ and Ar² can be the same or different and are each independently aryl or aryl substituted at one or a plurality of positions with halogen, nitro, nitroso, lower alkyl, aryl or substituted aryl, lower alkoxy, lower alkoxyalkyl, or cycloalkyl or cycloalkyl alkoxy, where each cycloalkyl group has from 3-7 members, where up to two of the cycloalkyl members are optionally hetero atoms selected from sulfur, oxygen and nitrogen, and where any member of the alkyl, aryl or cycloalkyl group is optionally substituted with halogen, lower alkyl or lower alkoxy, aryl or substituted aryl, halogen, nitro, nitroso, aldehyde, carboxylic acid, amide, ester, or sulfate, and
optionally Ar¹ and Ar² may be cojoined to create a tricyclic scaffold, 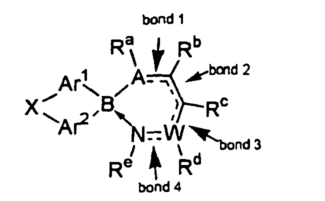 where X = C=O, CHOH, (CH₂)ₙ (n = 0 to 2), -CH=CH-, NR^{f} (R^{f} = H, C₁-C₄ alkyl, phenyl, thienyl, or pyridyl), O, SOₙ (n = 0 to 2), which have a plurality of positions with halogen, nitro, nitroso, lower alkyl, aryl or substituted aryl, lower alkoxy, lower alkoxyalkyl, or cycloalkyl or cycloalkyl alkoxy, where each cycloalkyl group has from 3-7 members, where up to two of the cycloalkyl members are optionally hetero atoms selected from sulfur, oxygen and nitrogen, and
wherein bond 1, bond 2, bond 3 and bond 4 are independently a single bond or a double bond, provided that when A is S or O, bond 1 is a single bond and where A is N, bond 1 is a double bond
with the proviso that if A is O, X is not present, and Ar¹ and Ar² are not cojoined, then p is not 0.

2. Use of a therapeutically-effective amount of a compound set forth in the formula of Claim 1 for preparing a medicament for the treatment of a disease or disorder associated with infection with a pathogenic bacteria that expresses an adenine DNA methyltransferase.

3. The Use according to Claim 2 wherein the pathogenic bacteria is *Staphylococcus aureus; Staphylococcus saprophyticus; Streptococcus pyrogenes; Streptococcus agalactie ; Streptococcus pneumonlae; Bacillus anthracis; Corynebacterium diphtheria; Clostridium perfringens; Clostridium botulinum; Clostridium tetani; Neisseria gonorrhoeae; Neisseria meningitidis; Pseudomonas aeruginosa; Legionella pneumophila; Escherichia coli; Yersifzia pestis; Hemophilus influenzae; Helicobacter pylori; Campylobacter fetus; Vibrio cholerae; Vibrio parahemolyticus; Trepomena pallidum; Actinomyces israelii; Rickettsia prowazekii; Rickettsia rickettsii; Chlamydia trachomatis; Chlamydia psittaci; Brucella abortus; Agrobacterium tumefaciens* or *Bacillus subtilis.*

4. A combinatorial library comprising a multiplicity of compounds set forth in the formula of claim 1.

5. A packaged pharmaceutical composition comprising the pharmaceutical composition of Claims 1 in a container and instructions for using the composition to treat a patient suffering from a disease or disorder associated with infection with a pathogenic bacterium.

6. The pharmaceutical composition for use in humans according to claim 1,
wherein
A is N, O or S;
W is Cₚ, where p is 0 or 1;
R^{a}, R^{b}, R^{c}, R^{d}, and R^{e} are the same or different and are independently hydrogen, halogen, lower alkyl, aryl that is phenyl, biphenyl, 1,2,3,4-tetrahydronaphthyl, naphthyl, anthryl, or phenanthryl, wherein the aryl groups are optionally substituted with 1, 2, or 3 groups that are selected from halogen, lower alkyl, lower alkoxy, lower alkylthio, trifluoromethyl, lower acyloxy, phenyl, biphenyl, 1,2,3,4-tetrahydronaphthyl, naphthyl, anthryl, phenanthryl, thienyl, furanyl, thiazolyl, imidazolyl, (is)oxazolyl, pyridyl, pyrimidinyl, (iso)quinolinyl, napthyridinyl, benzimidazolyl, benzoxazolyl and hydroxy, lower alkoxy, lower alkoxyalkyl, cycloalkyl or cycloalkyl alkoxy, where each cycloalkyl group has from 3-7 members, where up to two of the cycloalkyl members are optionally hetero atoms selected from sulfur, oxygen and nitrogen, and where any member of the alkyl, aryl or cycloalkyl group is optionally substituted with halogen, lower alkyl or lower alkoxy, aryl selected from phenyl, biphenyl, 1,2,3,4-tetrahydronaphthyl, naphthyl, anthryl, and phenanthryl or substituted aryl, wherein the aryl group is optionally substituted with 1, 2, or 3 groups that are selected from halogen, lower alkyl, lower alkoxy, lower alkylthio, trifluoromethyl, lower acyloxy, phenyl, biphenyl, 1,2,3,4-tetrahydronaphthyl, naphthyl, anthryl, phenanthryl, thienyl, furanyl, thiazolyl, imidazolyl, (is)oxazolyl, pyridyl, pyrimidinyl, (iso)quinolinyl, napthyridinyl, benzimidazolyl, benzoxazolyl and hydroxy, halogen, nitro, nitroso, aldehyde, carboxylic acid, amide, ester, or sulfate, or
wherein R^{a}, R^{b}, R^{c}, R^{d}, and R^{e} may be connected by aromatic selected from phenyl, biphenyl, 1,2,3,4-tetrahydronaphthyl, naphthyl, anthryl, and phenanthryl, aliphatic, heteroaromatic selected from thienyl, furanyl, thiazolyl, imidazolyl, (is)oxazolyl, pyridyl, pyrimidinyl, (iso)quinolinyl, napthyridinyl, benzimidazolyl, and benzoxazolyl, heteroaliphatic selected from piperidinyl, piperazinyl, morpholinyl, pyrrolidinyl, imidazolidinyl, oxazolidinyl, azaperhydropinyl, oxazaperhydropinyl, oxepanyl, oxazaperhydropinyl, and oxadiazaperhydropinyl ring structures or substituted embodiments thereof, where R^{a} is absent when A is O or S and R^{d} is absent when p = 0; and
Ar¹ and Ar² can be the same or different and are each independently phenyl, biphenyl, 1,2,3,4-tetrahydronaphthyl, naphthyl, anthryl, and phenanthryl or phenyl, biphenyl, 1,2,3,4-tetrahydronaphthyl, naphthyl, anthryl, and phenanthryl substituted at one or a plurality of positions with halogen, nitro, nitroso, lower alkyl, phenyl, biphenyl, 1,2,3,4-tetrahydronaphthyl, naphthyl, anthryl, and phenanthryl, lower alkoxy, lower alkoxyalkyl, or cycloalkyl or cycloalkyl alkoxy, where each cycloalkyl group has from 3-7 members, where up to two of the cycloalkyl members are optionally hetero atoms selected from sulfur, oxygen and nitrogen, and where any member of the alkyl, aryl or cycloalkyl group is optionally substituted with halogen, lower alkyl or lower alkoxy, aryl selected from phenyl, biphenyl, 1,2,3,4-tetrahydronaphthyl, naphthyl, anthryl, and phenanthryl or phenyl, biphenyl, 1,2,3,4-tetrahydronaphthyl, naphthyl, anthryl, and phenanthryl substituted with 1, 2, or 3 groups that are selected from halogen, lower alkyl, lower alkoxy, lower alkylthio, trifluoromethyl, lower acyloxy, phenyl, biphenyl, 1,2,3,4-tetrahydronaphthyl, naphthyl, anthryl, phenanthryl, thienyl, furanyl, thiazolyl, imidazolyl, (is)oxazolyl, pyridyl, pyrimidinyl, (iso)quinolinyl, napthyridinyl, benzimidazolyl, benzoxazolyl and hydroxy, halogen, nitro, nitroso, aldehyde, carboxylic acid, amide, ester, or sulfate, and optionally
Ar¹ and Ar² may be cojoined to create a tricyclic scaffold, where X = C=O, CHOH, (CH₂)ₙ (n = 0 to 2), -CH=CH-, NR^{f} (R^{f} = H, C₁-C₄ alkyl, phenyl, thienyl, or pyridyl), O, SOₙ (n = 0 to 2), which have a plurality of positions with halogen, nitro, nitroso, lower alkyl, aryl selected from phenyl, biphenyl, 1,2,3,4-tetrahydronaphthyl, naphthyl, anthryl, and phenanthryl or aryl substituted with 1, 2, or 3 groups that are selected from halogen, lower alkyl, lower alkoxy, lower alkylthio, trifluoromethyl, lower acyloxy, phenyl, biphenyl, 1,2,3,4-tetrahydronaphthyl, naphthyl, anthryl, phenanthryl, thienyl, furanyl, thiazolyl, imidazolyl, (is)oxazolyl, pyridyl, pyrimidinyl, (iso)quinolinyl, napthyridinyl, benzimidazolyl, benzoxazolyl and hydroxy, lower alkoxy, lower alkoxyalkyl, or cycloalkyl or cycloalkyl alkoxy, where each cycloalkyl group has from 3-7 members, where up to two of the cycloalkyl members are optionally hetero atoms selected from sulfur, oxygen and nitrogen, and 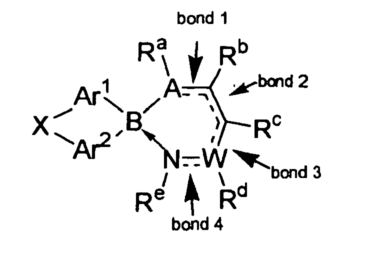 wherein bond 1, bond 2, bond 3 and bond 4 are independently a single bond or a double bond, provided that when A is S or O, bind 1 is a single bond and where A is N, bond 1 is a double bond.

7. The pharmaceutical composition according to claim 6, wherein
R^{a}, R^{b}, R^{c}, R^{d}, and R^{e} are the same or different and are independently hydrogen, halogen, lower alkyl, optionally substituted phenyl or naphthyl, wherein the phenyl and naphthyl, are optionally substituted with 1, 2, or 3 groups that are selected from halogen, lower alkyl, lower alkoxy, lower alkylthio, trifluoromethyl, lower acyloxy, phenyl, naphthyl, thiazolyl, pyrimidinyl, pyridyl, and hydroxy, lower alkoxy, lower alkoxyalkyl, or cycloalkyl or cycloalkyl alkoxy, where each cycloalkyl group has from 3-7 members, where up to two of the cycloalkyl members are optionally hetero atoms selected from sulfur, oxygen and nitrogen, and where any member of the alkyl, aryl or cycloalkyl group is optionally substituted with halogen, lower alkyl or lower alkoxy, aryl selected from phenyl and naphthyl, wherein the aryl group is optionally substituted with 1, 2, or 3 groups that are selected from halogen, lower alkyl, lower alkoxy, lower alkylthio, trifluoromethyl, lower acyloxy, phenyl, naphthyl, thiazolyl, pyrimidinyl, pyridyl, and hydroxy, halogen, nitro, nitroso, aldehyde, carboxylic acid, amide, ester, or sulfate, or
wherein R^{a}, R^{b}, R^{c}, R^{d}, and R^{e} may be connected by aromatic selected from phenyl, and naphthyl, aliphatic, heteroaromatic selected from thiazolyl, pyrimidinyl, and pyridyl, heteroaliphatic selected from piperidinyl, piperazinyl, morpholinyl, pyrrolidinyl, imidazolidinyl, oxazolidinyl, azaperhydropinyl, oxazaperhydropinyl, oxepanyl, oxazaperhydropinyl, and oxadiazaperhydropinyl ring structures or substituted embodiments thereof, where R^{a} is absent when A is O or S and R^{d} is absent when p = 0; and
Ar¹ and Ar² can be the same or different and are each independently phenyl, or naphthyl, substituted at one or a plurality of positions with halogen, nitro, nitroso, lower alkyl, phenyl, naphthyl, lower alkoxy, lower alkoxyalkyl, or cycloalkyl or cycloalkyl alkoxy, where each cycloalkyl group has from 3-7 members, where up to two of the cycloalkyl members are optionally hetero atoms selected from sulfur, oxygen and nitrogen, and where any member of the alkyl, aryl or cycloalkyl group is optionally substituted with halogen, lower alkyl or lower alkoxy, aryl selected from phenyl, and naphthyl, wherein the
phenyl, and naphthyl, are optionally substituted with 1, 2, or 3 groups that are selected from halogen, lower alkyl, lower alkoxy, lower alkylthio, trifluoromethyl, lower acyloxy, phenyl, naphthyl, thiazolyl, pyrimidinyl, pyridyl and hydroxy, halogen, nitro, nitroso, aldehyde, carboxylic acid, amide, ester, or sulfate,
and optionally
Ar¹ and Ar² may be cojoined to create a tricyclic scaffold, where X = C=O, CHOH, (CH₂)ₙ (n = 0 to 2), -CH=CH-, NR^{f} (R^{f} = H, C₁-C₄ alkyl, phenyl, thienyl, or pyridyl), O, SOₙ (n = 0 to 2), which have a plurality of positions with halogen, nitro, nitroso, lower alkyl, aryl selected from phenyl, and naphthyl, wherein the phenyl and naphthyl groups are optionally substituted with 1, 2, or 3 groups that are selected from halogen, lower alkyl, lower alkoxy, lower alkylthio, trifluoromethyl, lower acyloxy, phenyl, naphthyl, thienyl, pyridyl, pyrimidinyl, and hydroxy, lower alkoxy, lower alkoxyalkyl, or cycloalkyl or cycloalkyl alkoxy, where each cycloalkyl group has from 3-7 members, where up to two of the cycloalkyl members are optionally hetero atoms selected from sulfur, oxygen and nitrogen, and 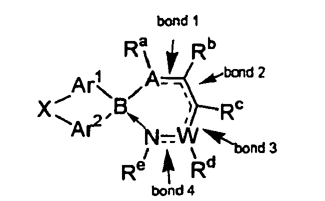 wherein bond 1, bond 2, bond 3 and bond 4 are independently a single bond or a double bond, provided that when A is S or O, bind 1 is a single bond and where A is N, bond 1 is a double bond.

8. The pharmaceutical composition according to claim 6, wherein
R^{a}, R^{b}, R^{c}, R^{d}, and R^{e} are the same or different and are independently hydrogen, halogen, lower alkyl, optionally substituted phenyl or naphthyl, wherein the phenyl and naphthyl are optionally substituted with 1, 2, or 3 groups that are selected from halogen, lower alkyl, lower alkoxy, lower alkylthio, trifluoromethyl, lower acyloxy, phenyl, naphthyl, 1-imidazolyl, 2-thienyl, 1-, or 2- quinolinyl, 1-, or 2- isoquinolinyl, 1-, or 2- tetrahydro isoquinolinyl, 2- or 3-furanyl and 2- tetrahydrofuranyl, and hydroxy, lower alkoxy, lower alkoxyalkyl, or cycloalkyl or cycloalkyl alkoxy, where each cycloalkyl group has from 3-7 members, where up to two of the cycloalkyl members are optionally hetero atoms selected from sulfur, oxygen and nitrogen, and where any member of the alkyl, aryl or cycloalkyl group is optionally substituted with halogen, lower alkyl or lower alkoxy, aryl selected from phenyl, and naphthyl, or substituted aryl, wherein the aryl group is optionally substituted with 1, 2, or 3 groups that are selected from halogen, lower alkyl, lower alkoxy, lower alkylthio, trifluoromethyl, lower acyloxy, phenyl, naphthyl, 1-imidazolyl, 2-thienyl, 1-, or 2- quinolinyl, 1-, or 2- isoquinolinyl, 1-, or 2- tetrahydro isoquinolinyl, 2- or 3-furanyl and 2- tetrahydrofuranyl, and hydroxy, halogen, nitro, nitroso, aldehyde, carboxylic acid, amide, ester, or sulfate, or
wherein R^{a}, R^{b}, R^{c}, R^{d}, and R^{e} may be connected by aromatic selected from phenyl, and naphthyl, aliphatic, heteroaromatic selected 1-imidazolyl, 2-thienyl, 1-, or 2- quinolinyl, 1-, or 2- isoquinolinyl, 1-, or 2- tetrahydro isoquinolinyl, 2- or 3-furanyl and 2- tetrahydrofuranyl, heteroaliphatic selected from piperidinyl, piperazinyl, morpholinyl, pyrrolidinyl, imidazolidinyl, oxazolidinyl, azaperhydropinyl, oxazaperhydropinyl, oxepanyl, oxazaperhydropinyl, and oxadiazaperhydropinyl ring structures or substituted embodiments thereof, where R^{a} is absent when A is O or S and R^{d} is absent when p = 0; and
Ar¹ and Ar² can be the same or different and are each independently phenyl, or naphthyl, substituted at one or a plurality of positions with halogen, nitro, nitroso, lower alkyl, phenyl, naphthyl, lower alkoxy, lower alkoxyalkyl, or cycloalkyl or cycloalkyl alkoxy, where each cycloalkyl group has from 3-7 members, where up to two of the cycloalkyl members are optionally hetero atoms selected from sulfur, oxygen and nitrogen, and where any member of the alkyl, aryl or cycloalkyl group is optionally substituted with halogen, lower alkyl or lower alkoxy, aryl selected from phenyl, and naphthyl, wherein the phenyl, and naphthyl, are optionally substituted with 1, 2, or 3 groups that are selected from halogen, lower alkyl, lower alkoxy, lower alkylthio, trifluoromethyl, lower acyloxy, phenyl, naphthyl, 1-imidazolyl, 2-thienyl, 1-, or 2- quinolinyl, 1-, or 2- isoquinolinyl, 1-, or 2- tetrahydro isoquinolinyl, 2- or 3-furanyl and 2- tetrahydrofuranyl and hydroxy, halogen, nitro, nitroso, aldehyde, carboxylic acid, amide, ester, or sulfate,
and optionally
Ar¹ and Ar² may be cojoined to create a tricyclic scaffold, where X = C=O, CHOH, (CH₂)ₙ (n = 0 to 2), -CH=CH-, NR^{f} (R^{f} = H, C₁-C₄ alkyl, phenyl, thienyl, or pyridyl), O, SOₙ (n = 0 to 2), which have a plurality of positions with halogen, nitro, nitroso, lower alkyl, aryl selected from phenyl, and naphthyl, wherein the phenyl and naphthyl groups are optionally substituted with 1, 2, or 3 groups that are selected from halogen, lower alkyl, lower alkoxy, lower alkylthio, trifluoromethyl, lower acyloxy, phenyl, naphthyl, 1-imidazolyl, 2-thienyl, 1-, or 2- quinolinyl, 1-, or 2- isoquinolinyl, 1-, or 2- tetrahydro isoquinolinyl, 2- or 3-furanyl 2- tetrahydrofuranyl, and hydroxy, lower alkoxy, lower alkoxyalkyl, or cycloalkyl or cycloalkyl alkoxy, where each cycloalkyl group has from 3-7 members, where up to two of the cycloalkyl members are optionally hetero atoms selected from sulfur, oxygen and nitrogen, and 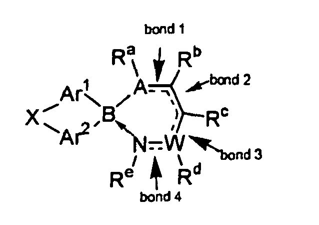 wherein bond 1, bond 2, bond 3 and bond 4 are independently a single bond or a double bond, provided that when A is S or O, bind 1 is a single bond and where A is N, bond 1 is a double bond.

9. The pharmaceutical composition according to claim 7, wherein
Ar¹ and Ar² are both phenyl, each of which is optionally substituted at one or a plurality of positions with halogen, nitro, nitroso, lower alkyl, phenyl, naphthyl, lower alkoxy, lower alkoxyalkyl, or cycloalkyl or cycloalkyl alkoxy, where each cycloalkyl group has from 3-7 members, where up to two of the cycloalkyl members are optionally hetero atoms selected from sulfur, oxygen and nitrogen, and where any member of the alkyl, aryl or cycloalkyl group is optionally substituted with halogen, lower alkyl or lower alkoxy, aryl selected from phenyl and naphthyl, wherein the phenyl and naphthyl, are optionally substituted with 1, 2, or 3 groups that are selected from halogen, lower alkyl, lower alkoxy, lower alkylthio, trifluoromethyl, lower acyloxy, phenyl, naphthyl, thiazolyl, pyrimidinyl, pyridyl and hydroxy, halogen, nitro, nitroso, aldehyde, carboxylic acid, amide, ester, or sulfate.

10. The pharmaceutical composition according to claim 8, wherein
Ar¹ and Ar² are both phenyl, each of which is optionally substituted at one or a plurality of positions with halogen, nitro, nitroso, lower alkyl, phenyl, naphthyl, lower alkoxy, lower alkoxyalkyl, or cycloalkyl or cycloalkyl alkoxy, where each cycloalkyl group has from 3-7 members, where up to two of the cycloalkyl members are optionally hetero atoms selected from sulfur, oxygen and nitrogen, and where any member of the alkyl, aryl or cycloalkyl group is optionally substituted with halogen, lower alkyl or lower alkoxy, aryl selected from phenyl, and naphthyl, wherein the phenyl, and naphthyl, are optionally substituted with 1, 2, or 3 groups that are selected from halogen, lower alkyl, lower alkoxy, lower alkylthio, trifluoromethyl, lower acyloxy, phenyl, naphthyl, 1-imidazolyl, 2-thienyl, 1-, or 2- quinolinyl, 1-, or 2- isoquinolinyl, 1-, or 2-tetrahydro isoquinolinyl, 2- or 3- furanyl and 2- tetrahydrofuranyl and hydroxy, halogen, nitro, nitroso, aldehyde, carboxylic acid, amide, ester, or sulfate.

11. The pharmaceutical composition according to any of claims 6, 8 and 10, wherein
R^{a}, R^{b}, R^{c}, R^{d}, and R^{e} are connected by an aliphatic or heteroaromatic group selected from thienyl, furanyl, thiazolyl, imidazolyl, (is)oxazolyl, pyridyl, pyrimidinyl, (iso)quinolinyl, napthyridinyl, benzimidazolyl, and benzoxazolyl, or a substituted embodiments thereof.

12. The pharmaceutical composition according to claim 11, wherein
R^{a}, R^{b}, R^{c}, R^{d} and R^{e} are connected by heteroaromatic group selected from pyridyl and quinolinyl, or substituted embodiments thereof.

13. The use of any of claims 2 or 3, wherein the compound set forth in the formula of Claim 1 is specified as set forth in any of claims 6-12.

## Patentansprüche

1. Eine pharmazeutische Zusammensetzung zur Verwendung im Menschen, welche eine Verbindung der folgenden Formel umfasst: 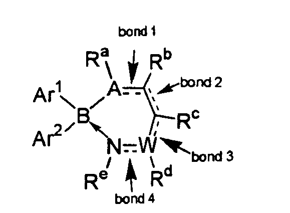 einschließend alle pharmazeutisch verträglichen Salze von solchen Verbindungen und zumindest einen pharmazeutisch verträglichen Träger oder Hilfsstoff;
wobei A = N, O oder S ist;
W = Cₚ ist, wobei p = 0 oder 1 ist;
R^{a}, R^{b}, R^{c}, R^{d}, und R^{e} gleich oder unterschiedlich sind und unabhängig voneinander Wasserstoff, Halogen, niedriges Alkyl, Aryl oder substituiertes Aryl, niedriges Alkoxy, niedriges Alkoxyaryl, oder Cycloalkyl oder Cycloalkylalkoxy sind, wobei jede Cykloalkylgruppe von 3- 7 Gliedern aufweist, wobei bis zu zwei der Cycloalkylglieder optional Heteroatome sind, ausgewählt aus Schwefel, Sauerstoff und Stickstoff, und wobei irgendein Glied der Alkyl-, Aryl- oder Cycloalkyl- Gruppe optional substitutiert ist mit Halogen, niedrigem Alkyl oder niedrigem Alkoxy, Aryl oder substituiertem Aryl, Halogen, Nitro, Nitroso, Aldehyd, Carbonsäure, Amid, Ester, oder Sulfat, oder wobei R^{a}, R^{b}, R^{c}, R^{d}, und R^{e} verknüpft sein können durch aromatische, aliphatische, heteroaromatische, heteroaliphatische Ringstrukturen oder substitutierte Ausführungsformen davon, wobei R^{a} fehlt, wenn A gleich O oder S ist und R^{d} fehlt, wenn p = 0 ist; und
wobei Ar¹ und Ar² gleich oder unterschiedlich sein können und jeweils unabhängig voneinander Aryl oder Aryl, substituiert an zumindest einer oder einer Vielzahl von Positonen mit Halogen, Nitro, Nitroso, niedrigem Alkyl, Aryl oder substituiertem Aryl, niedrigem Alkoxy, niedrigem Alkoxyalkyl, oder Cycloalkyl oder Cycloalkylalkoxy sind, wobei jede Cycloalkylgruppe von 3-7 Gliedern aufweist, wobei bis zu zwei der Cycloalkylglieder optional Heteroatome sind, ausgewählt aus Schwefel, Sauerstoff und Stickstoff, und wobei irgendein Glied der Alkyl- , Aryl- oder Cycloalkyl- Gruppe optional substitutiert ist mit Halogen, niedrigem Alkyl oder niedrigem Alkoxy, Aryl oder substituiertem Aryl, Halogen, Nitro, Nitroso, Aldehyd, Carbonsäure, Amid, Ester, oder Sulfat, und
optional Ar¹ und Ar² miteinander verbunden sein können, um eine tricyclische Form zu erzeugen, 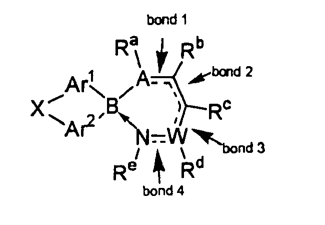 wobei X = C=O, CHOH, (CH₂)ₙ (n = 0 to 2), - CH=CH- , NR^{f} (R^{f} = H, C₁- C₄ Alkyl, Phenyl, Thienyl, oder Pyridyl), O, SOₙ (n = 0 bis 2), welche eine Vielzahl von Positionen aufweisen mit Halogen, Nitro, Nitroso, niedrigem Alkyl, Aryl oder substituiertem Aryl, niedrigem Alkoxy, niedrigem Alkoxyalkyl, oder Cycloalkyl oder Cycloalkylalkoxy, wobei jede Cycloalkylgruppe von 3- 7 Glieder aufweist, wobei bis zu zwei der Cycloalkyl Glieder optional Heteroatome sind, die ausgewählt sind aus Schwefel, Sauerstoff und Stickstoff, und
wobei Bindung 1, Bindung 2, Bindung 3 und Bindung 4 unabhängig voneinander eine Einfachbindung oder eine Doppelbindung darstellen, vorausgesetzt, dass, wenn A gleich S oder O ist, Bindung 1 eine Einfachbindung ist und wenn A gleich N ist, Bindung 1 eine Doppelbindung ist,
unter der Maßgabe, dass falls A gleich O ist, X nicht vorliegt und Ar¹ und Ar² nicht miteinander verbunden sind, dann p nicht 0 ist.

2. Verwendung einer therapeutisch effektiven Menge einer Verbindung, dargestellt in der Formel von Anspruch 1, zum Herstellen eines Medikaments für die Behandlung einer Erkrankung oder eines Leidens, assoziiert mit einer Infektion mit einem pathogenen Bakterium, welches eine Adenin- DNA- Methyltransferase exprimiert.

3. Die Verwendung gemäß Anspruch 2, wobei das pathogene Bakterium ein *Staphylococcus aureus; Staphylococcus saprophyticus; Streptococcus pyrogenes; Streptococcus agalactie ; Streptococcus pneumonlae; Bacillus anthracis; Coderynebacterium diphtheria; Clostridium perfringens; Clostridium botulinum; Clostridium tetani; Neisseria gonoderrhoeae; Neisseria meningitidis; Pseudomonas aeruginosa;Legionella pneumophila; Escherichia coli; Yersifzia pestis; Hemophilus influenzae; Helicobacter pylori; Campylobacter fetus; Vibrio cholerae; Vibrio parahemolyticus; Trepomena pallidum; Actinomyces israelii; Rickettsia prowazekii; Rickettsia rickettsii; Chlamydia trachomatis; Chlamydia psittaci; Brucella abortus; Agrobacterium tumefaciens* oder *Bacillus subtilis* ist.

4. Eine kombinatorische Bibliothek umfassend eine Vielzahl von Verbindungen, dargestellt in der Formel von Anspruch 1.

5. Eine verpackte pharmazeutische Zusammensetzung umfassend die pharmazeutische Zusammensetzung von Anspruch 1 in einem Behälter sowie Instruktionen zur Verwendung zur Zusammensetzung, um einen Patienten zu behandeln, welcher unter einer Erkrankung oder einem Leiden, assoziiert mit einer Infektion mit einem pathogenen Bakterium leidet.

6. Die pharmazeutische Zusammensetzung zur Anwendung im Menschen gemäß Anspruch 1, wobei
A = N, O oder S ist;
W = Cₚ ist, wobei p = 0 oder 1 ist;
R^{a}, R^{b}, R^{c}, R^{d}, und R^{e} gleich sind, oder unterschiedlich voneinander und unabhängig voneinander Wasserstoff, Halogen, niedriges Alkyl, Aryl, welches Phenyl, Biphenyl, 1,2,3,4- tetrahydronaphthyl, Naphthyl, Anthryl, oder Phenanthryl ist, sind, wobei die Arylgruppen optional substituiert sind mit 1, 2, oder 3 Gruppen, welche ausgewählt sind aus Halogen, niedrigem Alkyl, niedrigem Alkoxy, niedrigem Alkylthio, Trifluoromethyl, niedrigem Acyloxy, Phenyl, Biphenyl, 1,2,3,4- Tetrahydronaphthyl, Naphthyl, Anthryl, Phenanthryl, Thienyl, Furanyl, Thiazolyl, Imidazolyl, (Is)oxazolyl, Pyridyl, Pyrimidinyl, (Iso)quinolinyl, Napthyridinyl, Benzimidazolyl, Benzoxazolyl und Hydroxy, niedrigem Alkoxy, niedrigem Alkoxyalkyl, Cycloalkyl oder Cycloalkylalkoxy, wobei jede Cycloalkylgruppe von 3- 7 Gliedern aufweist, wobei bis zu zwei der Cycloalkylglieder optional Heteroatome sind, ausgewählt aus Schwefel, Sauerstoff und Stickstoff, und wobei irgendein Glied der Alkyl- , Aryl- oder Cycloalkylgruppen optional substituiert ist mit Halogen, niedrigem Alkyl oder niedrigem Alkoxy, Aryl ausgewählt aus Phenyl, Biphenyl, 1,2,3,4- Tetrahydronaphthyl, Naphthyl, Anthryl, und Phenanthryl, oder substituiertes Aryl, wobei die Arylgruppe optional substituiert ist mit 1, 2, oder 3 Gruppen, welche ausgewählt sind aus Halogen, niedrigem Alkyl, niedrigem Alkoxy, niedrigem Alkylthio, Trifluoromethyl, niedrigem Acyloxy, Phenyl, Biphenyl, 1,2,3,4- Tetrahydronaphthyl, Naphthyl, Anthryl, Phenanthryl, Thienyl, Furanyl, Tiazolyl, Imidazolyl, (is)oxazolyl, Pyridyl, Pyrimidinyl, (Iso)quinolinyl, Napthyridinyl, Benzimidazolyl, Benzoxazolyl und Hydroxy, Halogen, Nitro, Nitroso, Aldehyde, Carbonsäure, Amid, Ester, oder Sulfat, oder
wobei R^{a}, R^{b}, R^{c}, R^{d}, und R^{e} verknüpft sein können durch Aromaten ausgewählt aus Phenyl, Biphenyl, 1,2,3,4- Tetrahydronaphthyl, Naphthyl, Anthryl, und Phenanthryl, Aliphaten, Heteroaromaten ausgewählt aus Thienyl, Furanyl, Thiazolyl, Imidazolyl, (Is)oxazolyl, Pyridyl, Pyrimidinyl, (Iso)quinolinyl, Napthyridinyl, Benzimidazolyl, und Benzoxazolyl, Heteroaliphaten, ausgewählt aus Piperidinyl, Piperazinyl, Morpholinyl, Pyrrolidinyl, Imidazolidinyl, Oxazolidinyl, Azaperhydropinyl, Oxazaperhydropinyl, Oxepanyl, Oxazaperhydropinyl, und Oxadiazaperhydropinyl- Ringstrukturen, oder substituierten Ausführungsformen davon, wobei R^{a} fehlt, wenn A gleich O oder S ist und R^{d} fehlt, wenn p = 0 ist; und
Ar¹ und Ar² gleich sein können oder unterschiedlich und jeweils unabhängig voneinander Phenyl, Biphenyl, 1,2,3,4- Tetrahydronaphthyl, Naphthyl, Anthryl, und Phenanthryl sind oder Phenyl, Biphenyl, 1,2,3,4- Tetrahydronaphthyl, Naphthyl, Anthryl, und Phenanthryl, substituiert an einer oder einer Vielzahl von Positionen mit Halogen, Nitro, Nitroso, niedrigem Alkyl, Phenyl, Biphenyl, 1,2,3,4- Tetrahydronaphthyl, Naphthyl, Anthryl, und Phenanthryl, niedrigem Alkoxy, niedrigem Alkoxyalkyl, oder Cycloalkyl oder Cycloalkylalkoxy, wobei jede Cycloalkylgruppe von 3- 7 Glieder aufweist, wobei bis zu zwei der Cycloalkylglieder optional Heteroatome sind, ausgewählt aus Schwefel, Sauerstoff und Stickstoff, und wobei irgendein Glied der Alkyl- , Aryl- oder Cycloalkylgruppe optional substituiert ist mit Halogen, niedrigem Alkyl oder niedrigem Alkoxy, Aryl, ausgewählt aus Phenyl, Biphenyl, 1,2,3,4- Tetrahydronaphthyl, Naphthyl, Anthryl, und Phenanthryl oder Phenyl, Biphenyl, 1,2,3,4- tetrahydronaphthyl, Naphthyl, Anthryl, und Phenanthryl, substituiert mit 1, 2, oder 3 Gruppen, welche ausgewählt sind aus Halogen, niedrigem Alkyl, niedrigem Alkoxy, niedrigem Alkylthio, Trifluoromethyl, niedrigem Acyloxy, Phenyl, Biphenyl, 1,2,3,4-Tetrahydronaphthyl, Naphthyl, Anthryl, Phenanthryl, Thienyl, Furanyl, Thiazolyl, Imidazolyl, (Is)oxazolyl, Pyridyl, Pyrimidinyl, (Iso)quinolinyl, Napthyridinyl, Benzimidazolyl, Benzoxazolyl und Hydroxy, Halogen, Nitro, Nitroso, Aldehyde, Carbonsäure, Amid, Ester, oder Sulfat, und optional
Ar¹ und Ar² miteinander verbunden sein können, um eine tricyclische Form auszubilden, wobei X = C=O, CHOH, (CH₂)ₙ (n = 0 to 2), - CH=CH- , NR^{f} (R^{f} = H, C₁- C₄ Alkyl, Phenyl, Thienyl, oder Pyridyl), O, SOₙ (n = 0 to 2), welche eine Vielzahl von Positionen aufweisen mit Halogen, Nitro, Nitroso, niedrigem Alkyl, Aryl ausgewählt aus Phenyl, Biphenyl, 1,2,3,4-tetrahydronaphthyl, Naphthyl, Anthryl, und Phenanthryl oder Aryl substituiert mit 1, 2, oder 3 Gruppen, welche ausgewählt sind aus Halogen, niedrigem Alkyl, niedrigem Alkoxy, niedrigem Alkylthio, Trifluoromethyl, niedrigem Acyloxy, Phenyl, Biphenyl, 1,2,3,4- tetrahydronaphthyl, Naphthyl, Anthryl, Phenanthryl, Thienyl, Furanyl, Thiazolyl, Imidazolyl, (Is)oxazolyl, Pyridyl, Pyrimidinyl, (Iso)quinolinyl, Napthyridinyl, Benzimidazolyl, Benzoxazolyl und Hydroxy, niedrigem Alkoxy, niedrigem Alkoxyalkyl, oder Cycloalkyl oder Cycloalkyl Alkoxy, wobei jede Cycloalkylgruppe von 3- 7 Gliedern aufweist, wobei bis zu zwei der Cycloalkylglieder optional Heteroatome sind, ausgewählt aus Schwefel, Sauerstoff und Stickstoff und 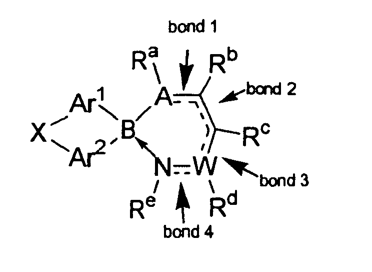 wobei Bindung 1, Bindung 2, Bindung 3 und Bindung 4 unabhängig voneinander eine Einfachbindung oder eine Doppelbindung darstellen, vorausgesetzt, dass, wenn A gleich S oder O ist, die Bindung 1 eine Einfachbindung ist und wenn A gleich N ist, Bindung 1 eine Doppelbindung ist.

7. Die pharmazeutische Zusammensetzung entsprechend Anspruch 6, wobei
R^{a}, R^{b}, R^{c}, R^{d}, und R^{e} gleich oder unterschiedlich sind und unabhängig voneinander Wasserstoff, Halogen, niedriges Alkyl, optional substituiertes Phenyl oder Naphthyl sind, wobei das Phenyl bzw. Naphthyl optional substituiert ist mit 1, 2, oder 3 Gruppen, welche ausgewählt sind aus Halogen, niedrigem Alkyl, niedrigem Allkoxy, niedrigem Alkylthio, Trifluoromethyl, niedrigem Acyloxy, Phenyl, Naphthyl, Thiazolyl, Pyrimidinyl, Pyridyl, und Hydroxy, niedrigem Alkoxy, niedrigem Alkoxyalkyl, oder Cycloalkyl oder Cycloalkylalkoxy, wobei jede Cycloalkylgruppe von 3- 7 Glieder aufweist, wobei bis zu zwei der Cycloalkylglieder optional Heteroatome sind, ausgewählt aus Schwefel, Sauerstoff und Stickstoff, und wobei irgendein Glied der Alkyl-, Aryl- oder Cycloalkylgruppe optional substituiert ist mit Halogen, niedrigem Alkyl oder niedrigem Alkoxy, Aryl ausgewählt aus Phenyl und Naphthyl, wobei die Arylgruppe optional substituiert ist mit 1, 2, oder 3 Gruppen, welche ausgewählt aus Halogen, niedrigem Alkyl, niedrigem Alkoxy, niedrigem Alkylthio, Trifluoromethyl, niedrigem Acyloxy, Phenyl, Naphthyl, Thiazolyl, Pyrimidinyl, Pyridyl, und Hydroxy, Halogen, Nitro, Nitroso, Aldehyd, Carboxylsäure, Amid, Ester, oder Sulfat, oder
wobei R^{a}, R^{b}, R^{c}, R^{d}, und R^{e} verknüpft sein können durch Aromaten ausgewählt aus Phenyl und Naphthyl, Aliphaten, Heteroaromaten, ausgewählt aus Thiazolyl, Pyrimidinyl, und Pyridyl, Heteroaliphaten, ausgewählt aus Piperidinyl, Piperazinyl, Morpholinyl, Pyrrolidinyl, Imidazolidinyl, Oxazolidinyl, Azaperhydropinyl, Oxazaperhydropinyl, Oxepanyl, Oxazaperhydropinyl, und Oxadiazaperhydropinyl- Ringstrukturen oder substituierten Ausführungsformen davon, wobei R^{a} fehlt, wenn A gleich O oder S ist und R^{d} fehlt, wenn p = 0 ist; und
Ar¹ und Ar² gleich oder unterschiedlich sein können und jeweils unabhängig voneinander Phenyl oder Naphthyl sind, substituiert an einer oder einer Vielzahl von Positionen mit Halogen, Nitro, Nitroso, niedrigem Alkyl, Phenyl, Naphthyl, niedrigem Alkoxy, niedrigem Alkoxyalkyl, oder Cycloalkyl oder Cycloalkylalkoxy, wobei jede Cycloalkylgruppe 3- 7 Glieder aufweist, wobei bis zu zwei der Cycloalkylglieder optional Heteroatome sind, ausgewählt aus Schwefel, Sauerstoff und Stickstoff, und wobei irgendein Glied der Alkyl, Aryl oder Cycloalkyl- Gruppe optional substituiert ist mit Halogen, niedrigem Alkyl oder niedrigem Alkoxy, Aryl ausgewählt aus Phenyl, und Naphthyl, wobei das Phenyl, und Naphthyl, optional substituiert ist mit 1, 2, oder 3 Gruppen, welche ausgewählt sind aus Halogen, niedrigem Alkyl, niedrigem Alkoxy, niedrigem Alkylthio, Trifluoromethyl, niedrigem Acyloxy, Phenyl, Naphthyl, Thiazolyl, Pyrimidinyl, Pyridyl und Hydroxy, Halogen, Nitro, Nitroso, Aldehyde, Carbonsäure, Amid, Ester, oder Sulfat,
und optional
Ar¹ und Ar² miteinander verbunden sein können, um eine tricyclische Form zu erzeugen, wobei X = C=O, CHOH, (CH₂)ₙ (n = 0 to 2), - CH=CH-, NR^{f} (R^{f} = H, C₁- C₄ Alkyl, Phenyl, Thienyl, oder Pyridyl), O, SOₙ (n = 0 to 2), welche eine Vielzahl von Positionen aufweisen mit Halogen, Nitro, Nitroso, niedrigem Alkyl, Aryl ausgewählt aus Phenyl und Naphthyl, wobei das Phenyl- bzw. Naphthyl optional substituiert sind mit 1, 2, oder 3 Gruppen, welche ausgewählt sind aus Halogen, niedrigem Alkyl, niedrigem Alkoxy, niedrigem Alkylthio, Trifluoromethyl, niedrigem Acyloxy, Phenyl, Naphthyl, Thienyl, Pyridyl, Pyrimidinyl und Hydroxy, niedrigem Alkoxy, niedrigem Alkoxyalkyl, oder Cycloalkyl oder Cycloalkylalkoxy, wobei jede Cycloalkylgruppe von 3- 7 Gliedern aufweist, wobei bis zu zwei der Cycloalkylglieder optional Heteroatome ausgewählt aus Schwefel, Sauerstoff und Stickstoff, sind und 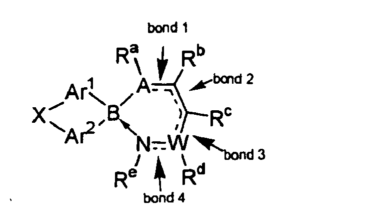 wobei Bindung 1, Bindung 2, Bindung 3 und Bindung 4 unabhängig voneinander eine Einfachbindung oder eine Doppelbindung darstellen, vorausgesetzt dass, wenn A gleich S oder O ist, Bindung 1 eine Einfachbindung ist und wenn A gleich N ist, Bindung 1 eine Doppelbindung ist.

8. Die pharmazeutische Zusammensetzung entsprechend Anspruch 6, wobei
R^{a}, R^{b}, R^{c}, R^{d}, und R^{e} gleich oder unterschiedlich sind und unabhängig voneinander Wasserstoff, Halogen, niedriges Alkyl, optional substituiertes Phenyl oder Naphthyl sind, wobei das Phenyl bzw. Naphthyl optional substituiert ist mit 1, 2, oder 3 Gruppen, welche ausgewählt sind aus Halogen, niedrigem Alkyl, niedrigem Alkoxy, niedrigem Alkylthio, Trifluoromethyl, niedrigem Acyloxy, Phenyl, Naphthyl, 1-Imidazolyl, 2- Thienyl, 1-, oder 2- Quinolinyl, 1- , oder 2- Isoquinolinyl, 1- , oder 2- Tetrahydroisoquinolinyl, 2- oder 3- Furanyl und 2- Tetrahydrofuranyl, und Hydroxy, niedrigem Alkoxy, niedrigem Alkoxyalkyl, oder Cycloalkyl oder Cycloalkyl Alkoxy, wobei jede Cycloalkylgruppe von 3- 7 Glieder aufweist, wobei bis zu zwei der Cycloalkylglieder optional Heteroatome sind, ausgewählt aus Schwefel, Sauerstoff und Stickstoff, wobei jedes Glied der Alkyl-, Aryl- oder Cycloalkylgruppe optional substituiert ist mit Halogen, niedrigem Alkyl oder niedrigem Alkoxy, Aryl ausgewählt aus Phenyl und Naphthyl, oder substituiertem Aryl, wobei die Arylgruppe optional substituiert ist mit 1, 2, oder 3 Gruppen, welche ausgewählt sind aus Halogen, niedrigem Alkyl, niedrigem Alkoxy, niedrigem Alkylthio, Trifluoromethyl, niedrigem Acyloxy, Phenyl, Naphthyl, 1- Imidazolyl, 2- Thienyl, 1- , oder 2- Quinolinyl, 1- , oder 2-Isoquinolinyl, 1- , oder 2- Tetrahydroisoquinolinyl, 2- oder 3- Furanyl und 2- Tetrahydrofuranyl, und Hydroxy, Halogen, Nitro, Nitroso, Aldehyd, Carbonsäure, Amid, Ester, oder Sulfat, oder
wobei R^{a}, R^{b}, R^{c}, R^{d}, und R^{e} verknüpft sein können durch Aromaten, ausgewählt aus Phenyl und Naphthyl, Aliphaten, Heteroaromaten ausgewählt aus 1-Imidazolyl, 2- Thienyl, 1- , oder 2- Quinolinyl, 1- , oder 2- Isoquinolinyl, 1-, oder 2- Tetrahydroisoquinolinyl, 2- oder 3- Furanyl und 2- Tetrahydrofuranyl, Heteroaliphaten, ausgewählt aus Piperidinyl, Piperazinyl, Morpholinyl, Pyrrolidinyl, Imidazolidinyl, Oxazolidinyl, Azaperhydropinyl, Oxazaperhydropinyl, Oxepanyl, Oxazaperhydropinyl, und Oxadiazaperhydropinyl- Ringstrukturen, oder substituierten Ausführungsformen davon, wobei R^{a} fehlt, wenn A gleich O oder S ist und R^{d} fehlt, wenn p = 0 ist; und
Ar¹ und Ar² die gleichen oder unterschiedlich sein können und jeweils unabhängig voneinander Phenyl, oder Naphthyl sind, substituiert an einer oder einer Vielzahl von Positionen mit Halogen, Nitro, Nitroso, niedrigem Alkyl, Phenyl, Naphthyl, niedrigem Alkoxy, niedrigem Alkoxyalkyl, oder Cycloalkyl oder Cycloalkyl Alkoxy, wobei jede Cycloalkylgruppe von 3- 7 Gliedern aufweist, wobei bis zu zwei der Cycloalkylglieder optional Heteroatome sind, ausgewählt aus Schwefel, Sauerstoff und Stickstoff und wobei irgendein Glied der Alkyl- , Aryl- oder Cycloalkylgruppe optional substituiert ist mit Halogen, niedrigem Alkyl oder niedrigem Alkoxy, Aryl ausgewählt aus Phenyl, und Naphthyl, wobei das Phenyl bzw. Naphthyl optional substituiert ist mit 1, 2, oder 3 Gruppen, welche ausgewählt sind aus Halogen, niedrigem Alkyl, niedrigem Alkoxy, niedrigem Alkylthio, Trifluoromethyl, niedrigem Acyloxy, Phenyl, Naphthyl, 1- Imidazolyl, 2- Thienyl, 1- , oder 2-Quinolinyl, 1-, oder 2- Isoquinolinyl, 1-, oder 2- Tetrahydroisoquinolinyl, 2- oder 3- Furanyl und 2- Tetrahydrofuranyl und Hydroxy, Halogen, Nitro, Nitroso, Aldehyde, Carbonsäure, Amid, Ester, oder Sulfat und optional
Ar¹ und Ar² miteinander verbunden sein können, um eine tricyclische Form zu erzeugen, wobei X = C=O, CHOH, (CH₂)ₙ (n = 0 to 2), - CH=CH- , NR^{f} (R^{f} = H, C₁- C₄ Alkyl, Phenyl, Thienyl, oder Pyridyl), O, SOₙ (n = 0 to 2) sind, welche eine Vielzahl von Positionen aufweisen mit Halogen, Nitro, Nitroso, niedrigem Alkyl, Aryl ausgewählt aus Phenyl und Naphthyl, wobei die Phenyl- bzw. Naphthyl- Gruppe optional substituiert ist mit 1, 2, oder 3 Gruppen, welche ausgewählt sind aus Halogen, niedrigem Alkyl, niedrigem Alkoxy, niedrigem Alkylthio, Trifluoromethyl, niedrigem Acyloxy, Phenyl, Naphthyl, 1- Imidazolyl, 2- Thienyl, 1- , oder 2- Quinolinyl, 1- , oder 2-Isoquinolinyl, 1- , oder 2- Tetrahydroisoquinolinyl, 2- oder 3- Furanyl 2-tetrahydrofuranyl, und Hydroxy, niedrigem Alkoxy, niedrigem Alkoxyalkyl, oder Cycloalkyl oder Cycloalkyl Alkoxy, wobei jede Cycloalkylgruppe von 3- 7 Gliedern aufweist, wobei bis zu zwei der Cycloalkylglieder optional Heteroatome ausgewählt aus Schwefel, Sauerstoff und Stickstoff sind, und 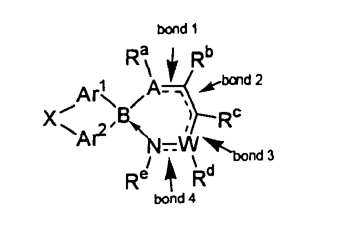 wobei Bindung 1, Bindung 2, Bindung 3 und Bindung 4 unabhängig voneinander eine Einfachbindung oder eine Doppelbindung darstellen, vorausgesetzt dass, wenn A gleich S oder O ist, Bindung 1 eine Einfachbindung ist und wenn A gleich N ist, Bindung 1 eine Doppelbindung ist.

9. Die pharmazeutische Zusammensetzung entsprechend Anspruch 7, wobei Ar¹ und Ar² beide Phenyl sind, wobei jedes davon optional substituiert ist an einer oder einer Vielzahl von Positionen mit Halogen, Nitro, Nitroso, niedrigem Alkyl, Phenyl, Naphthyl, niedrigem Alkoxy, niedrigem Alkoxyalkyl, oder Cycloalkyl oder Cycloalkylalkoxy, wobei jede Cycloalkylgruppe von 3- 7 Gliedern aufweist, wobei bis zu zwei der Cycloalkylglieder optional Heteroatome sind, ausgewählt aus Schwefel, Sauerstoff und Stickstoff, und wobei irgendein Glied der Alkyl- , Aryl- oder Cycloalkylgruppe optional substituiert ist mit Halogen, niedrigem Alkyl oder niedrigem Alkoxy, Aryl ausgewählt aus Phenyl, und Naphthyl, wobei das Phenyl bzw. Naphthyl optional substituiert ist mit 1, 2, oder 3 Gruppen, welche ausgewählt sind aus Halogen, niedrigem Alkyl, niedrigem Alkoxy, niedrigem Alkylthio, Trifluoromethyl, niedrigem Acyloxy, Phenyl, Naphthyl, Thiazolyl, Pyrimidinyl, Pyridyl und Hydroxy, Halogen, Nitro, Nitroso, Aldehyd, Carbonsäure, Amid, Ester oder Sulfat.

10. Die pharmazeutische Zusammensetzung entsprechend Anspruch 8, wobei Ar¹ und Ar² beide Phenyl sind, wobei jedes davon optional substituiert ist an einer oder einer Vielzahl von Positionen mit Nitro, Nitroso, niedrigem Alkyl, Phenyl, Naphthyl, niedrigem Alkoxy, niedrigem Alkoxyalkyl, oder Cycloalkyl oder Cycloalkyl Alkoxy, wobei jede Cycloalkylgruppe von 3- 7 Gliedern aufweist, wobei bis zu zwei der Cycloalkylglieder optional Heteroatome sind, ausgewählt aus Schwefel, Sauerstoff und Stickstoff, und wobei irgendein Glied der Alkyl- , Aryl- oder Cycloalkylgruppe optional substituiert ist mit Halogen, niedrigem Alkyl oder niedrigem Alkoxy, Aryl ausgewählt aus Phenyl, und Naphthyl, wobei das Phenyl bzw. Naphthyl optional substituiert ist mit 1, 2, oder 3 Gruppen, welche ausgewählt sind aus Halogen, niedrigem Alkyl, niedrigem Alkoxy, niedrigem Alkylthio, Trifluoromethyl, niedrigem Acyloxy, Phenyl, Naphthyl, 1- Imidazolyl, 2- Thienyl, 1- , oder 2-Quinolinyl, 1- , oder 2- Isoquinolinyl, 1- , oder 2- Tetrahydroisoquinolinyl, 2- oder 3-Furanyl und 2- Tetrahydrofuranyl, und Hydroxy, Halogen, Nitro, Nitroso, Aldehyd, Carbonsäure, Amid, Ester oder Sulfat.

11. Die pharmazeutische Zusammensetzung gemäß irgendeinem der Ansprüche 6, 8 oder 10, wobei R^{a}, R^{b}, R^{c}, R^{d}, und R^{e} verknüpft sind durch eine aliphatische oder hetero aromatische Gruppe, ausgewählt aus Thienyl, Furanyl, Thiazolyl, Imidazolyl, (Is)oxazolyl, Pyridyl, Pyrimidinyl, (Iso)quinolinyl, Napthyridinyl, Benzimidazolyl, und Benzoxazolyl, oder substituierte Ausführungsformen davon.

12. Die pharmazeutische Zusammensetzung gemäß Anspruch 11, wobei R^{a}, R^{b}, R^{c}, R^{d}, und R^{e} verknüpft sind durch eine aliphatische oder heteroaromatische Gruppe, ausgewählt aus Thienyl, Furanyl, Thiazolyl, Imidazolyl, (Is)oxazolyl, Pyridyl, Pyrimidinyl, (I-so)quinolinyl, Napthyridinyl, Benzimidazolyl, und Benzoxazolyl, oder substituierte Ausführungsformen davon.

13. Die Verwendung von irgendeinem der Ansprüche 2 oder 3, wobei die Verbindung, dargestellt in der Formel von Anspruch 1, spezifiziert ist, wie dargestellt in irgendeinem der Ansprüche 6 bis 12.

## Revendications

1. Composition pharmaceutique pour une utilisation chez les hommes, comprenant un composé de formule : 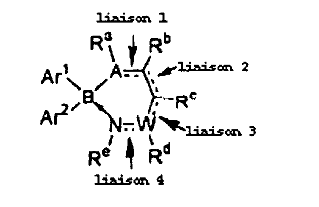 y compris tous les sels pharmaceutiquement acceptables de tels composés, et au moins un support ou un excipient pharmaceutiquement acceptable ;
où A représente N, O ou S ;
W représente Cₚ, où p vaut 0 ou 1 ;
R^{a}, R^{b}, R^{c}, R^{d} et R^{e} sont identiques ou différents et représentent indépendamment un atome d'hydrogène, d'halogène, un groupe alkyle inférieur, aryle ou aryle substitué, alcoxy inférieur, alcoxyalkyle inférieur, ou cycloalkyle ou cycloalkylalcoxy, où chaque groupe cycloalkyle a de 3 à 7 chaînons, où jusqu'à deux des chaînons du groupe cycloalkyle sont éventuellement des hétéroatomes choisis parmi le soufre, l'oxygène et l'azote, et où un chaînon quelconque du groupe alkyle, aryle ou cycloalkyle est éventuellement substitué par un atome d'halogène, un groupe alkyle inférieur ou alcoxy inférieur, aryle ou aryle substitué, halogène, nitro, nitroso, aldéhyde, acide carboxylique, amide, ester ou sulfate, ou bien
où R^{a}, R^{b}, R^{c}, R^{d} et R^{e} peuvent être reliés par des structures cycliques aromatiques, aliphatiques, hétéroaromatiques, hétéroaliphatiques ou par des modes de réalisation substitués de celles-ci, où R^{a} est absent lorsque A représente O ou S et R^{d} est absent lorsque p = 0 ; et
où Ar¹ et Ar² peuvent être identiques ou différents et représentent chacun indépendamment un groupe aryle ou aryle substitué au niveau d'une position ou d'une pluralité de positions par un atome d'halogène, un groupe nitro, nitroso, alkyle inférieur, aryle ou aryle substitué, alcoxy inférieur, alcoxyalkyle inférieur, ou cycloalkyle ou cycloalkylalcoxy, où chaque groupe cycloalkyle a de 3 à 7 chaînons, où jusqu'à deux des chaînons du groupe cycloalkyle sont éventuellement des hétéroatomes choisis parmi le soufre, l'oxygène et l'azote, et où un membre quelconque du groupe alkyle, aryle ou cycloalkyle est éventuellement substitué par un atome d'halogène, un groupe alkyle inférieur ou alcoxy inférieur, aryle ou aryle substitué, halogène, nitro, nitroso, aldéhyde, acide carboxylique, amide, ester ou sulfate, et
éventuellement Ar¹ et Ar² peuvent être reliés pour créer une structure tricyclique, 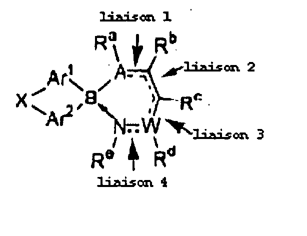 où X = C=O, CHOH, (CH₂)ₙ (n = 0 à 2), -CH=CH-, NR^{f} (R^{f} = H, alkyle en C₁ en C₄, phényle, thiényle ou pyridyle), O, SOₙ (n = 0 à 2), qui présentent une pluralité de positions avec un atome d'halogène, un groupe nitro, nitroso, alkyle inférieur, aryle ou aryle substitué, alcoxy inférieur, alcoxyalkyle inférieur, ou cycloalkyle ou cycloalkylalcoxy, où chaque groupe cycloalkyle a de 3 à 7 chaînons, où jusqu'à deux des chaînons du groupe cycloalkyle sont éventuellement des hétéroatomes choisis parmi le soufre, l'oxygène et l'azote, et
où liaison 1, liaison 2, liaison 3 et liaison 4 représentent indépendamment une liaison simple ou une double liaison, à condition que lorsque A représente S ou O, la liaison 1 soit une simple liaison et où A représente N, la liaison 1 soit une double liaison.
à condition que si A représente O, X ne soit pas présent, et que si Ar¹ et Ar² ne sont pas reliés, alors p ne vaille pas 0.

2. Utilisation d'une quantité thérapeutiquement efficace d'un composé représenté par la formule de la revendication 1 pour la préparation d'un médicament destiné au traitement d'une maladie ou d'un trouble associé à une infection par une bactérie pathogène qui exprime une adénine ADN méthyltransférase.

3. Utilisation selon la revendication 2, où la bactérie pathogène est *Staphylococcus aureus* ; *Staphylococcus saprophyticus; Streptococcus pyrogenes ; Streptococcus agalactiae ; Streptococcus pneumoniae ; Bacillus anthracis ; Corynebacterium diphtheriae ; Clostridium perfringens ; Clostridium botulinum ; Clostridium tetani ; Neisseria gonorrhoeae ; Neisseria meningitidis ; Pseudomonas aeruginosa ; Legionella pneumophila ; Escherichia coli ; Yersinia pestis ; Hemophilus influenzae* ; *Helicobacter pylori ; Campylobacter fetus ; Vibrio cholerae ; Vibrio parahemolyticus ; Treponema pallidum ; Actinomyces israelii ; Rickettsia prowazekii ; Rickettsia rickettsii ; Chlamydia trachomatis ; Chlamydia psittaci ; Brucella abortus ; Agrobacterium tumefaciens* ou *Bacillus subtilis.*

4. Banque combinatoire comprenant une multiplicité de composés représentés par la formule de la revendication 1.

5. Composition pharmaceutique conditionnée comprenant la composition pharmaceutique selon la revendication 1 dans un récipient et des instructions d'utilisation de la composition pour traiter un patient souffrant d'une maladie ou d'un trouble associé à une infection par une bactérie pathogène.

6. Composition pharmaceutique pour une utilisation chez les hommes selon la revendication 1,
où
A représente N, O ou S ;
W représente Cₚ, où p vaut 0 ou 1 ;
R^{a}, R^{b}, R^{c}, R^{d} et R^{e} sont identiques ou différents et représentent indépendamment un atome d'hydrogène, d'halogène, un groupe alkyle inférieur, aryle qui est un groupe phényle, biphényle, 1,2,3,4-tétrahydro-naphtyle, naphtyle, anthryle ou phénanthryle, où les groupe aryle sont éventuellement substitués par 1, 2 ou 3 groupes qui sont choisis parmi un atome d'halogène, un groupe alkyle inférieur, alcoxy inférieur, alkyl(inférieur)thio, trifluorométhyle, acyloxy inférieur, phényle, biphényle, 1,2,3,4-tétrahydro-naphtyle, naphtyle, anthryle, phénanthryle, thiényle, furanyle, thiazolyle, imidazolyle, (is)oxazolyle, pyridyle, pyrimidinyle, (iso)quinoléinyle, napthyridinyle, benzimidazolyle, benzoxazolyle et hydroxy, alcoxy inférieur, alcoxyalkyle inférieur, ou cycloalkyle ou cycloalkylalcoxy, où chaque groupe cycloalkyle a de 3 à 7 chaînons, où jusqu'à deux des chaînons du groupe cycloalkyle sont éventuellement des hétéroatomes choisis parmi le soufre, l'oxygène et l'azote, et où un chaînon quelconque du groupe alkyle, aryle ou cycloalkyle est éventuellement substitué par un atome d'halogène, un groupe alkyle inférieur ou alcoxy inférieur, aryle choisi parmi un groupe phényle, biphényle, 1,2,3,4-tétrahydronaphtyle, naphtyle, anthryle et phénanthryle ou aryle substitué, où le groupe aryle est éventuellement substitué par 1, 2 ou 3 groupes qui sont choisis parmi un atome d'halogène, un groupe alkyle inférieur, alcoxy inférieur, alkyl(inférieur)thio, trifluorométhyle, acyloxy inférieur, phényle, biphényle, 1,2,3,4-tétrahydro-naphtyle, naphtyle, anthryle, phénanthryle, thiényle, furanyle, thiazolyle, imidazolyle, (is)oxazolyle, pyridyle, pyrimidinyle, (iso)quinoléinyle, napthyridinyle, benzimidazolyle, benzoxazolyle et hydroxy, halogène, nitro, nitroso, aldéhyde, acide carboxylique, amide, ester ou sulfate, ou bien
où R^{a}, R^{b}, R^{c}, R^{d} et R^{e} peuvent être reliés par des structures cycliques aromatiques choisies parmi un groupe phényle, biphényle, 1,2,3,4-tétrahydronaphtyle, naphtyle, anthryle et phénanthryle, aliphatiques, hétéroaromatiques choisies parmi un groupe thiényle, furanyle, thiazolyle, imidazolyle, (is)oxazolyle, pyridyle, pyrimidinyle, (iso)quinoléinyle, naphtyridinyle, benzimidazolyle et benzoxazolyle, hétéroaliphatiques choisies parmi un groupe pipéridinyle, pipérazinyle, morpholinyle, pyrrolidinyle, imidazolidinyle, oxazolidinyle, azaperhydropinyle, oxazaperhydropinyle, oxépanyle, oxazaperhydropinyle et oxadiazaperhydropinyle ou par des modes de réalisation substitués de celles-ci, où R^{a} est absent lorsque A représente O ou S et R^{d} est absent lorsque p = 0 ; et
Ar¹ et Ar² peuvent être identiques ou différents et représentent chacun indépendamment un groupe phényle, biphényle, 1,2,3,4-tétrahydronaphtyle, naphtyle, anthryle et phénanthryle ou un groupe phényle, biphényle, 1,2,3,4-tétrahydronaphtyle, naphtyle, anthryle et phénanthryle substitué au niveau d'une position ou d'une pluralité de positions par un atome d'halogène, un groupe nitro, nitroso, alkyle inférieur, phényle, biphényle, 1,2,3,4-tétrahydronaphtyle, naphtyle, anthryle et phénanthryle, alcoxy inférieur, alcoxyalkyle inférieur, ou cycloalkyle ou cycloalkylalcoxy, où chaque groupe cycloalkyle a de 3 à 7 chaînons, où jusqu'à deux des chaînons du groupe cycloalkyle sont éventuellement des hétéroatomes choisis parmi le soufre, l'oxygène et l'azote, et où un membre quelconque du groupe alkyle, aryle ou cycloalkyle est éventuellement substitué par un atome d'halogène, un groupe alkyle inférieur ou alcoxy inférieur, aryle choisi parmi un groupe phényle, biphényle, 1,2,3,4-tétrahydronaphtyle, naphtyle, anthryle et phénanthryle ou un groupe phényle, biphényle, 1,2,3,4-tétrahydronaphtyle, naphtyle, anthryle et phénanthryle substitué par 1, 2 ou 3 groupes qui sont choisis parmi un atome d'halogène, un groupe alkyle inférieur, alcoxy inférieur, alkyl(inférieur)thio, trifluorométhyle, acyloxy inférieur, phényle, biphényle, 1,2,3,4-tétrahydro-naphtyle, naphtyle, anthryle, phénanthryle, thiényle, furanyle, thiazolyle, imidazolyle, (is)oxazolyle, pyridyle, pyrimidinyle, (iso)quinoléinyle, napthyridinyle, benzimidazolyle, benzoxazolyle et hydroxy, halogène, nitro, nitroso, aldéhyde, acide carboxylique, amide, ester ou sulfate, et éventuellement
Ar¹ et Ar² peuvent être reliés pour créer une structure tricyclique, où X = C=O, CHOH, (CH₂)ₙ (n = 0 à 2), -CH=CH-, NR^{f} (R^{f} = H, alkyle en C₁ en C₄, phényle, thiényle ou pyridyle), O, SOₙ (n = 0 à 2), qui présentent une pluralité de positions avec un atome d'halogène, un groupe nitro, nitroso, alkyle inférieur, aryle choisi parmi un groupe phényle, biphényle, 1,2,3,4-tétrahydronaphtyle, naphtyle, anthryle et phénanthryle ou aryle substitué par 1, 2 ou 3 groupes qui sont choisis parmi un atome d'halogène, un groupe alkyle inférieur, alcoxy inférieur, alkyl(inférieur)-thio, trifluorométhyle, acyloxy inférieur, phényle, biphényle, 1,2,3,4-tétrahydro-naphtyle, naphtyle, anthryle, phénanthryle, thiényle, furanyle, thiazolyle, imidazolyle, (is)oxazolyle, pyridyle, pyrimidinyle, (iso)quinoléinyle, napthyridinyle, benzimidazolyle, benzoxazolyle et hydroxy, alcoxy inférieur, alcoxyalkyle inférieur, ou cycloalkyle ou cycloalkylalcoxy, où chaque groupe cycloalkyle a de 3 à 7 chaînons, où jusqu'à deux des chaînons du groupe cycloalkyle sont éventuellement des hétéroatomes choisis parmi le soufre, l'oxygène et l'azote, et 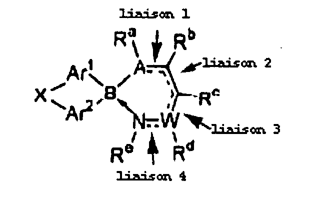 où liaison 1, liaison 2, liaison 3 et liaison 4 représentent indépendamment une liaison simple ou une double liaison, à condition que lorsque A représente S ou 0, la liaison 1 soit une simple liaison et où A représente N, la liaison 1 soit une double liaison.

7. Composition pharmaceutique selon la revendication 6, où
R^{a}, R^{b}, R^{c}, R^{d} et R^{e} sont identiques ou différents et représentent indépendamment un atome d'hydrogène, d'halogène, un groupe alkyle inférieur, phényle ou naphtyle éventuellement substitué, où les groupes phényle et naphtyle sont éventuellement substitués par 1, 2 ou 3 groupes qui sont choisis parmi un atome d'halogène, un groupe alkyle inférieur, alcoxy inférieur, alkyl(inférieur)thio, trifluorométhyle, acyloxy inférieur, phényle, naphtyle, thiazolyle, pyrimidinyle, pyridyle et hydroxy, alcoxy inférieur, alcoxyalkyle inférieur, ou cycloalkyle ou cycloalkylalcoxy, où chaque groupe cycloalkyle a de 3 à 7 chaînons, où jusqu'à deux des chaînons du groupe cycloalkyle sont éventuellement des hétéroatomes choisis parmi le soufre, l'oxygène et l'azote, et où un chaînon quelconque du groupe alkyle, aryle ou cycloalkyle est éventuellement substitué par un atome d'halogène, un groupe alkyle inférieur ou alcoxy inférieur, aryle choisi parmi un groupe phényle et naphtyle, où le groupe aryle est éventuellement substitué par 1, 2 ou 3 groupes qui sont choisis parmi un atome d'halogène, un groupe alkyle inférieur, alcoxy inférieur, alkyl (inférieur) thio, trifluorométhyle, acyloxy inférieur, phényle, naphtyle, thiazolyle, pyrimidinyle, pyridyle et hydroxy, halogène, nitro, nitroso, aldéhyde, acide carboxylique, amide, ester ou sulfate, ou bien
où R^{a}, R^{b}, R^{c}, R^{d} et R^{e} peuvent être reliés par des structures cycliques aromatiques choisies parmi un groupe phényle et naphtyle, aliphatiques, hétéroaromatiques choisies parmi un groupe thiazolyle, pyrimidinyle et pyridyle, hétéroaliphatiques choisies parmi un groupe pipéridinyle, pipérazinyle, morpholinyle, pyrrolidinyle, imidazolidinyle, oxazolidinyle, azaperhydropinyle, oxazaperhydropinyle, oxépanyle, oxazaperhydropinyle et oxadiazaperhydropinyle ou par des modes de réalisation substitués de celles-ci, où R^{a} est absent lorsque A représente O ou S et R^{d} est absent lorsque p = 0 ; et
Ar¹ et Ar² peuvent être identiques ou différents et représentent chacun indépendamment un groupe phényle ou naphtyle, substitué au niveau d'une position ou d'une pluralité de positions par un atome d'halogène, un groupe nitro, nitroso, alkyle inférieur, phényle, naphtyle, alcoxy inférieur, alcoxyalkyle inférieur, ou cycloalkyle ou cycloalkylalcoxy, où chaque groupe cycloalkyle a de 3 à 7 chaînons, où jusqu'à deux des chaînons du groupe cycloalkyle sont éventuellement des hétéroatomes choisis parmi le soufre, l'oxygène et l'azote, et où un membre quelconque du groupe alkyle, aryle ou cycloalkyle est éventuellement substitué par un atome d'halogène, un groupe alkyle inférieur ou alcoxy inférieur, aryle choisi parmi un groupe phényle et naphtyle, où les groupes phényle et naphtyle sont éventuellement substitués par 1, 2 ou 3 groupes qui sont choisis parmi un atome d'halogène, un groupe alkyle inférieur, alcoxy inférieur, alkyl(inférieur)-thio, trifluorométhyle, acyloxy inférieur, phényle, naphtyle, thiazolyle, pyrimidinyle, pyridyle et hydroxy, halogène, nitro, nitroso, aldéhyde, acide carboxylique, amide, ester ou sulfate,
et éventuellement
Ar¹ et Ar² peuvent être reliés pour créer une structure tricyclique, où X = C=O, CHOH, (CH₂)ₙ (n = 0 à 2), -CH=CH-, NR^{f} (R^{f} = H, alkyle en C₁ en C₄, phényle, thiényle ou pyridyle), O, SOₙ (n = 0 à 2), qui présentent une pluralité de positions avec un atome d'halogène, un groupe nitro, nitroso, alkyle inférieur, aryle choisi parmi un groupe phényle et naphtyle, où les groupes phényle et naphtyle sont éventuellement substitués par 1, 2 ou 3 groupes qui sont choisis parmi un atome d'halogène, un groupe alkyle inférieur, alcoxy inférieur, alkyl(inférieur)thio, trifluorométhyle, acyloxy inférieur, phényle, naphtyle, thiényle, pyridyle, pyrimidinyle et hydroxy, alcoxy inférieur, alcoxyalkyle inférieur, ou cycloalkyle ou cycloalkyl-alcoxy, où chaque groupe cycloalkyle a de 3 à 7 chaînons, où jusqu'à deux des chaînons du groupe cycloalkyle sont éventuellement des hétéroatomes choisis parmi le soufre, l'oxygène et l'azote, et 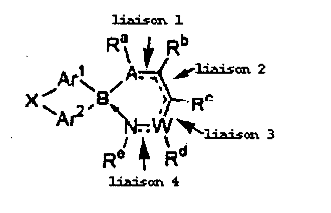 où liaison 1, liaison 2, liaison 3 et liaison 4 représentent indépendamment une liaison simple ou une double liaison, à condition que lorsque A représente S ou O, la liaison 1 soit une simple liaison et où A représente N, la liaison 1 soit une double liaison.

8. Composition pharmaceutique selon la revendication 6, où
R^{a}, R^{b}, R^{c}, R^{d} et R^{e} sont identiques ou différents et représentent indépendamment un atome d'hydrogène, d'halogène, un groupe alkyle inférieur, phényle ou naphtyle éventuellement substitué, où les groupes phényle et naphtyle sont éventuellement substitués par 1, 2 ou 3 groupes qui sont choisis parmi un atome d'halogène, un groupe alkyle inférieur, alcoxy inférieur, alkyl(inférieur)thio, trifluorométhyle, acyloxy inférieur, phényle, naphtyle, 1-imidazolyle, 2-thiényle, 1- ou 2-quinoléinyle, 1- ou 2-isoquinoléinyle, 1- ou 2-tétrahydroisoquinoléinyle, 2- ou 3-furanyle et 2-tétrahydrofuranyle et hydroxy, alcoxy inférieur, alcoxyalkyle inférieur, ou cycloalkyle ou cycloalkylalcoxy, où chaque groupe cycloalkyle a de 3 à 7 chaînons, où jusqu'à deux des chaînons du groupe cycloalkyle sont éventuellement des hétéroatomes choisis parmi le soufre, l'oxygène et l'azote, et où un chaînon quelconque du groupe alkyle, aryle ou cycloalkyle est éventuellement substitué par un atome d'halogène, un groupe alkyle inférieur ou alcoxy inférieur, aryle choisi parmi un groupe phényle et naphtyle, ou aryle substitué, où le groupe aryle est éventuellement substitué par 1, 2 ou 3 groupes qui sont choisis parmi un atome d'halogène, un groupe alkyle inférieur, alcoxy inférieur, alkyl(inférieur)thio, trifluorométhyle, acyloxy inférieur, phényle, naphtyle, 1-imidazolyle, 2-thiényle, 1- ou 2-quinoléinyle, 1- ou 2-isoquinoléinyle, 1- ou 2-tétrahydroisoquinoléinyle, 2- ou 3-furanyle et 2-tétrahydrofuranyle et hydroxy, halogène, nitro, nitroso, aldéhyde, acide carboxylique, amide, ester ou sulfate, ou bien
où R^{a}, R^{b}, R^{c}, R^{d} et R^{e} peuvent être reliés par des structures cycliques aromatiques choisies parmi un groupe phényle et naphtyle, aliphatiques, hétéroaromatiques choisies parmi un groupe 1-imidazolyle, 2-thiényle, 1- ou 2-quinoléinyle, 1- ou 2-isoquinoléinyle, 1- ou 2-tétrahydroisoquinoléinyle, 2- ou 3-furanyle et 2-tétrahydrofuranyle, hétéroaliphatiques choisies parmi un groupe pipéridinyle, pipérazinyle, morpholinyle, pyrrolidinyle, imidazolidinyle, oxazolidinyle, azaperhydropinyle, oxazaperhydropinyle, oxépanyle, oxazaperhydropinyle et oxadiazaperhydropinyle ou par des modes de réalisation substitués de celles-ci, où R^{a} est absent lorsque A représente 0 ou S et R^{d} est absent lorsque p = 0 ; et
Ar¹ et Ar² peuvent être identiques ou différents et représentent chacun indépendamment un groupe phényle ou naphtyle, substitué au niveau d'une position ou d'une pluralité de positions par un atome d'halogène, un groupe nitro, nitroso, alkyle inférieur, phényle, naphtyle, alcoxy inférieur, alcoxyalkyle inférieur, ou cycloalkyle ou cycloalkylalcoxy, où chaque groupe cycloalkyle a de 3 à 7 chaînons, où jusqu'à deux des chaînons du groupe cycloalkyle sont éventuellement des hétéroatomes choisis parmi le soufre, l'oxygène et l'azote, et où un membre quelconque du groupe alkyle, aryle ou cycloalkyle est éventuellement substitué par un atome d'halogène, un groupe alkyle inférieur ou alcoxy inférieur, aryle choisi parmi un groupe phényle et naphtyle, où les groupes phényle et naphtyle sont éventuellement substitués par 1, 2 ou 3 groupes qui sont choisis parmi un atome d'halogène, un groupe alkyle inférieur, alcoxy inférieur, alkyl(inférieur)-thio, trifluorométhyle, acyloxy inférieur, phényle, naphtyle, 1-imidazolyle, 2-thiényle, 1- ou 2-quinoléinyle, 1- ou 2-isoquinoléinyle, 1- ou 2-tétrahydroisoquinoléinyle, 2- ou 3-furanyle et 2-tétrahydrofuranyle et hydroxy, halogène, nitro, nitroso, aldéhyde, acide carboxylique, amide, ester ou sulfate,
et éventuellement
Ar¹ et Ar² peuvent être reliés pour créer une structure tricyclique, où X = C=O, CHOH, (CH₂)ₙ (n = 0 à 2), -CH=CH-, NR^{f} (R^{f} = H, alkyle en C₁ en C₄, phényle, thiényle ou pyridyle), O, SOₙ (n = 0 à 2), qui présentent une pluralité de positions avec un atome d'halogène, un groupe nitro, nitroso, alkyle inférieur, aryle choisi parmi un groupe phényle et naphtyle, où les groupes phényle et naphtyle sont éventuellement substitués par 1, 2 ou 3 groupes qui sont choisis parmi un atome d'halogène, un groupe alkyle inférieur, alcoxy inférieur, alkyl(inférieur)thio, trifluorométhyle, acyloxy inférieur, phényle, naphtyle, 1-imidazolyle, 2-thiényle, 1- ou 2-quinoléinyle, 1- ou 2-isoquinoléinyle, 1- ou 2-tétrahydroisoquinoléinyle, 2- ou 3-furanyle et 2-tétrahydrofuranyle et hydroxy, alcoxy inférieur, alcoxyalkyle inférieur, ou cycloalkyle ou cycloalkylalcoxy, où chaque groupe cycloalkyle a de 3 à 7 chaînons, où jusqu'à deux des chaînons du groupe cycloalkyle sont éventuellement des hétéroatomes choisis parmi le soufre, l'oxygène et l'azote, et 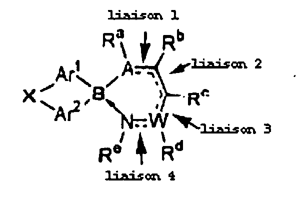 où liaison 1, liaison 2, liaison 3 et liaison 4 représentent indépendamment une liaison simple ou une double liaison, à condition que lorsque A représente S ou O, la liaison 1 soit une simple liaison et où A représente N, la liaison 1 soit une double liaison.

9. Composition pharmaceutique selon la revendication 7, où
Ar¹ et Ar² représentent tous les deux un groupe phényle, dont chacun est éventuellement substitué au niveau d'une position ou d'une pluralité de positions par un atome d'halogène, un groupe nitro, nitroso, alkyle inférieur, phényle, naphtyle, alcoxy inférieur, alcoxyalkyle inférieur, ou cycloalkyle ou cycloalkylalcoxy, où chaque groupe cycloalkyle a de 3 à 7 chaînons, où jusqu'à deux des chaînons du groupe cycloalkyle sont éventuellement des hétéroatomes choisis parmi le soufre, l'oxygène et l'azote, et où un membre quelconque du groupe alkyle, aryle ou cycloalkyle est éventuellement substitué par un atome d'halogène, un groupe alkyle inférieur ou alcoxy inférieur, aryle choisi parmi un groupe phényle et naphtyle, où les groupes phényle et naphtyle sont éventuellement substitués par 1, 2 ou 3 groupes qui sont choisis parmi un atome d'halogène, un groupe alkyle inférieur, alcoxy inférieur, alkyl(inférieur)-thio, trifluorométhyle, acyloxy inférieur, phényle, naphtyle, thiazolyle, pyrimidinyle, pyridyle et hydroxy, halogène, nitro, nitroso, aldéhyde, acide carboxylique, amide, ester ou sulfate.

10. Composition pharmaceutique selon la revendication 8, où
Ar¹ et Ar² représentent tous les deux un groupe phényle, dont chacun est éventuellement substitué au niveau d'une position ou d'une pluralité de positions par un atome d'halogène, un groupe nitro, nitroso, alkyle inférieur, phényle, naphtyle, alcoxy inférieur, alcoxyalkyle inférieur, ou cycloalkyle ou cycloalkylalcoxy, où chaque groupe cycloalkyle a de 3 à 7 chaînons, où jusqu'à deux des chaînons du groupe cycloalkyle sont éventuellement des hétéroatomes choisis parmi le soufre, l'oxygène et l'azote, et où un membre quelconque du groupe alkyle, aryle ou cycloalkyle est éventuellement substitué par un atome d'halogène, un groupe alkyle inférieur ou alcoxy inférieur, aryle choisi parmi un groupe phényle et naphtyle, où les groupes phényle et naphtyle sont éventuellement substitués par 1, 2 ou 3 groupes qui sont choisis parmi un atome d'halogène, un groupe alkyle inférieur, alcoxy inférieur, alkyl(inférieur)-thio, trifluorométhyle, acyloxy inférieur, phényle, naphtyle, 1-imidazolyle, 2-thiényle, 1- ou 2-quinoléinyle, 1- ou 2-isoquinoléinyle, 1- ou 2-tétrahydroisoquinoléinyle, 2- ou 3-furanyle et 2-tétrahydrofurànyle et hydroxy, halogène, nitro, nitroso, aldéhyde, acide carboxylique, amide, ester ou sulfate.

11. Composition pharmaceutique selon l'une quelconque des revendications 6, 8 et 10, où
R^{a}, R^{b}, R^{c}, R^{d} et R^{e} sont reliés par un groupe aliphatique ou hétéroaromatique choisi parmi un groupe thiényle, furanyle, thiazolyle, imidazolyle, (is)oxazolyle, pyridyle, pyrimidinyle, (iso)quinoléinyle, naphtyridinyle, benzimidazolyle et benzoxazolyle, ou des modes de réalisation substitués de ceux-ci.

12. Composition pharmaceutique selon la revendication 11, où
R^{a}, R^{b}, R^{c}, R^{d} et R^{e} sont reliés par un groupe hétéroaromatique choisi parmi un groupe pyridyle et quinoléinyle, ou des modes de réalisation substitués de ceux-ci.

13. Utilisation selon l'une quelconque des revendications 2 ou 3, où le composé représenté par la formule de la revendication 1 est spécifié comme il est présenté dans l'une quelconque des revendications 6 à 12.
